# EUROPÄISCHE PATENTANMELDUNG

(11) **EP 1 300 402 A1**
(43) Veröffentlichungstag der Anmeldung: **09.04.2003**
(21) Anmeldenummer: 02405830.7
(22) Anmeldetag: 26.09.2002
(51) Int. Cl.: C07D 295/096, C07C 217/58, C07D 213/38, C07D 295/185, A61L 2/18, A61K 7/50, A61P 31/02, A01N 33/10, A01N 43/36, A01N 43/40, A01N 43/84

(54) **Alkoxybenzylamine mit antimikrobiellen Eigenschaften**

(30) Priorität: 04.10.2001 EP 01810970
(71) Anmelder: Ciba Specialty Chemicals Holding Inc., 4057 Basel (CH)
(72) Erfinder: Haap, Wolfgang, 79639 Grenzach-Wyhlen (DE); Hölzl, Werner, 68440 Eschentzwiller (FR); Petzold, Karin, 79592 Fischingen (DE)

(57) **Zusammenfassung**

Beschrieben werden Alkoxybenzylamine der Formel 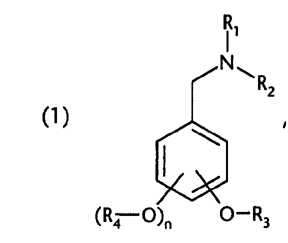 worin
- R₁ und R₂: unabhängig voneinander Wasserstoff, nicht substituiertes oder mit einem oder mehreren Hydroxy, Halogen, C₁-C₁₈-Alkyl, C₁-C₁₈-Alkoxy oder Amino substituiertes C₁-C₂₀-Alkyl, C₂-C₂₀-Alkenyl, C₂-C₂₀-Alkinyl, C₃-C₁₂-Cycloalkyl, C₃-C₇-Cycloalkyl-C₁-C₂₀-Alkyl, C₁-C₆-Alkoxy-C₁-C₂₀-Alkyl, C₁-C₁₂-Alkyl-oxycarbonyl, Phenyl, Phenyl-C₁-C₂₀-Alkyl; Carboxy; oder Pyridino-C₁-C₅-alkyl; oder
- R₁ und R₂: zusammen mit dem sie verbindenden Stickstoffatom einen 5- bis 7-gliedrigen monocyclischen heterocyclischen Ring bilden;
- R₃ und R₄: unabhängig voneinander nicht substituiertes oder mit einem oder mehreren Hydroxy, Halogen, C₁-C₁₈-Alkyl, C₁-C₁₈-Alkoxy, oder Amino substituiertes C₁-C₂₀-Alkyl; und
- n: 0 oder 1;
bedeuten.

Sie eignen sich zur antimikrobiellen Behandlung von Oberflächen, als antimikrobielle Wirksubstanz gegen grampositive und gramnegative Bakterien.

## Beschreibung

Die vorliegende Erfindung betrifft Alkoxybenzylamine, Herstellung dieser Verbindungen, sowie deren Verwendung zur antimikrobiellen Behandlung von Oberflächen, als antimikrobielle Wirksubstanz gegen grampositive und gramnegative Bakterien, Hefen und Pilze sowie zur Konservierung von Kosmetika, Haushaltsprodukten, Textilien, Kunststoffen und zur Verwendung in Desinfektionsmitteln.

Die erfindungsgemässen Alkoxybenzylamine entsprechen der Formel 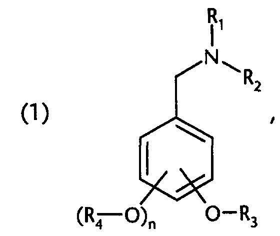 worin
- R₁ und R₂: unabhängig voneinander Wasserstoff, nicht substituiertes oder mit einem oder mehreren Hydroxy, Halogen, C₁-C₁₈-Alkyl, C₁-C₁₈-Alkoxy oder Amino substituiertes C₁-C₂₀-Alkyl, C₂-C₂₀-Alkenyl, C₂-C₂₀-Alkinyl, C₃-C₁₂-Cycloalkyl, C₃-C₇-Cycloalkyl-C₁-C₂₀-Alkyl, C₁-C₆-Alkoxy-C₁-C₂₀-Alkyl, C₁-C₁₂-Alkyl-oxycarbonyl, Phenyl, Phenyl-C₁-C₂₀-Alkyl; Carboxy; oder Pyridino-C₁-C₅-alkyl; oder
- R₁ und R₂: zusammen mit dem sie verbindenden Stickstoffatom einen 5- bis 7-gliedrigen monocyclischen heterocyclischen Ring bilden;
- R₃ und R₄: unabhängig voneinander nicht substituiertes oder mit einem oder mehreren Hydroxy, Halogen, C₁-C₁₈-Alkyl, C₁-C₁₈-Alkoxy, oder Amino substituiertes C₁-C₂₀-Alkyl; und
- n: 0 oder 1;
bedeuten.

C₁-C₂₀-Alkyl sind geradkettige oder verzweigte Alkylreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl, Pentadecyl, Hexadecyl, Heptadecyl, Octadecyl oder Eicosyl.

C₃-C₁₈-Cycloalkyl bedeutet z.B. Cyclopropyl, Cyclobutyl, Cyclopentyl, Cycloheptyl, Cyclooctyl, Cyclononyl, Cyclodecyl, Cycloundecyl, Cyclodocecyl, Cyclotetradecyl, Cyclopentadecyl, Cyclohexadecyl, Cycloheptadecyl, Cyclooctadecyl und insbesondere Cyclohexyl.

Alkenyl umfasst im Rahmen der angegebenen Bedeutungen u.a. Allyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Dodec-2-enyl oder n-Octadec-4-enyl.

C₁-C₅-Alkoxy sind geradkettige oder verzweigte Reste wie z.B. Methoxy, Ethoxy, Propoxy, Butoxy, Pentoxy, Heptyloxy, Octyloxy, Isooctyloxy, Nonyloxy, Decyloxy, Undecyloxy, Dodecyloxy, Tetradecyyloxy, Pentadecyloxy, Hexadecyloxy, Heptadecyloxy, Octadecyloxy oder Eicosyloxy.

Alkinyl umfasst z.B. Ethinyl, Propargyl, 2-Butinyl, 1 -Pentenyl oder 2-Pentenyl.

Bevorzugt sind Verbindungen der Formel (1), worin
- R₁ und R₂: unabhängig voneinander Wasserstoff; nicht substituiertes oder mit einem oder mehreren Hydroxy, Halogen, C₁-C₁₈-Alkyl, C₁-C₁₈-Alkoxy, Amino substituiertes C₁-C₂₀-Alkyl, Cyclo-C₅-C₇-Alkyl, Phenyl oder Phenyl-C₁-C₄-Alkyl; oder Pyridino-C₁-C₅-alkyl;
bedeuten.

Besonders bevorzugt sind dabei Verbindungen der Formel (1), worin
- R₁ und R₂: unabhängig voneinander C₁-C₂₀-Alkyl; Phenyl, Phenyl-C₁-C₄-Alkyl; oder Pyridino-C₁-C₄-Alkyl;
bedeuten, und insbesondere solche Verbindungen der Formel (1), worin
- R₁ und R₂: unabhängig voneinander C₁-C₁₂-Alkyl; Benzyl; oder Pyridinoethyl;
bedeuten.

Weiterhin sind Verbindungen der Formel (1) bevorzugt, worin
- R₁ und R₂: zusammen mit dem sie verbindenden Stickstoffatom einen nicht weiter substituierten oder mit einem oder mehreren C₁-C₅-Alkyl substituierten -(CH₂)₂₋₆- -Rest, der gegebenenfalls mit einem oder 2 -O- und/oder -NR'- -Gruppen und/oder - 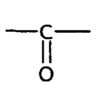 unterbrochen ist;
- R': Wasserstoff; nicht substituiertes oder mit einem oder mehreren Hydroxy, Halogen, C₁-C₁₈-Alkyl, C₁-C₁₈-Alkoxy, Amino oder quaternären Ammoniumugruppen substituiertes C₁-C₁₈-Alkyl, Cyclo-C₅-C₇-Alkyl oder Phenyl; -COR"; und
- R": Wasserstoff; oder C₁-C₄-Alkyl;
bedeuten.

Ganz besonders bevorzugt sind dabei Verbindungen der Formel (1), worin
- R': Wasserstoff; C₁-C₅-Alkyl; einen Rest der Formel 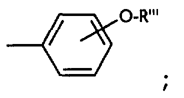 oder einen Rest der Formel 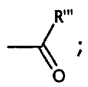 und
- R''': Wasserstoff; oder C₁-C₅-Alkyl;
bedeuten, und ganz besonders solche Verbindungen der Formel (1), worin
- R₃ und R₄: unabhängig voneinander C₁-C₁₈-Alkyl;
bedeuten.

Insbesondere sind Verbindungen der Formel (1) bevorzugt, worin
- R₃: C₃-C₁₂-Alkyl, und solche, worin
- R₄: Methyl
bedeutet.

Bevorzugt sind auch Verbindungen der Formel (1), worin R₃ und R₄ die gleiche Bedeutung haben.

Besonders bevorzugt sind Verbindungen der Formel 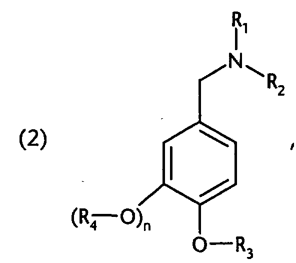 worin
R₁, R₂, R₃, R₄ und n die in Formel (1) angegebene Bedeutung haben.

Insbesondere sind Verbindungen der Formel (2), bevorzugt, worin
- R₁ und R₂: zusammen mit dem sie verbindenden Stickstoffatom einen nicht weiter substituierten oder mit einem oder mehreren C₁-C₅-Alkyl substituierten -(CH₂)₂₋₆- -Rest, der gegebenenfalls mit einem oder 2 -O- und/oder -NR'- -Gruppen und/oder 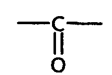 unterbrochen ist;
- R': Wasserstoff; nicht substituiertes oder mit einem oder mehreren Hydroxy, Halogen, C₁-C₁₈-Alkyl, C₁-C₁₈-Alkoxy, Amino oder quaternären Ammoniumugruppen substituiertes C₁-C₁₈-Alkyl, Cyclo-C₅-C₇-Alkyl oder Phenyl; -COR"; und
- R": Wasserstoff; oder C₁-C₄-Alkyl;
- R₃ und R₄: unabhängig voneinander C₁-C₁₈-Alkyl; und
- n: 0; oder 1;
bedeuten.

Weiterhin sind Verbindungen der Formel (2) bevorzugt, worin
- R₁ und R₂: unabhängig voneinander C₁-C₂₀-Alkyl; Phenyl; Phenyl-C₁-C₄-Alkyl; oder Pyridino-C₁-C₄-Alkyl;
- R₃: C₃-C₁₂-Alkyl; und
- R₄: C₁-C₁₂-Alkyl, insbesondere Methyl;
bedeuten.

In der nachfolgenden Tabelle 1 sind weitere erfindungsgemässe Alkoxybenzylamine beispielhaft aufgeführt:

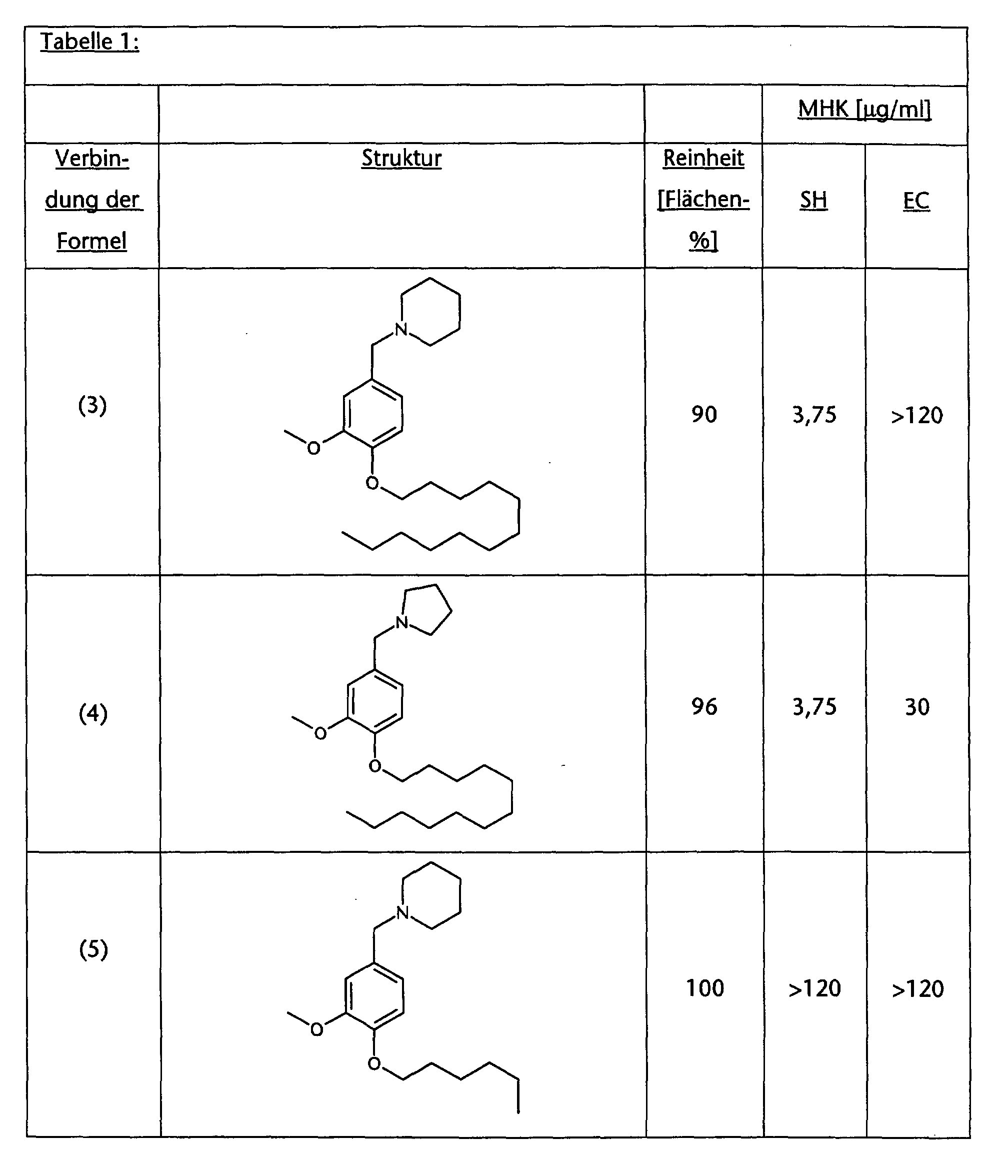

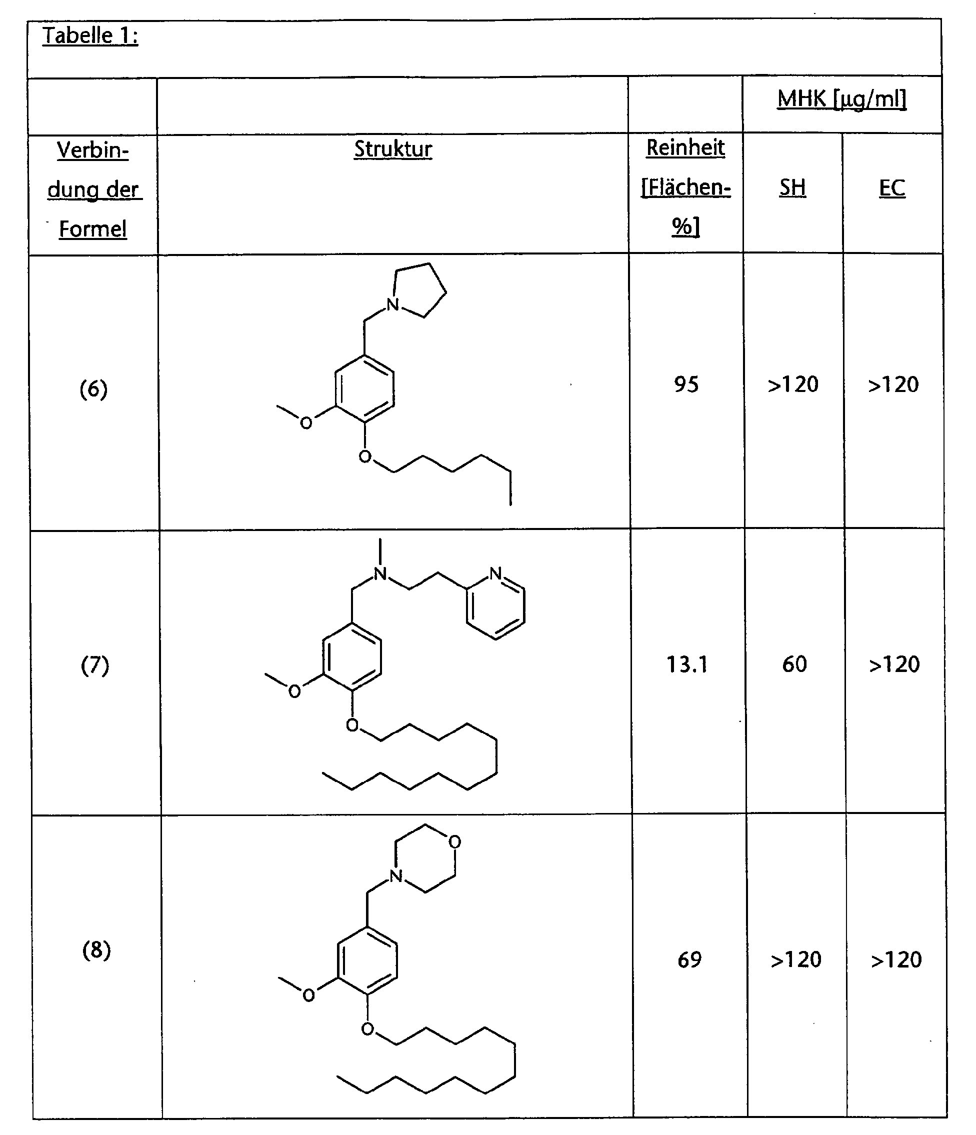

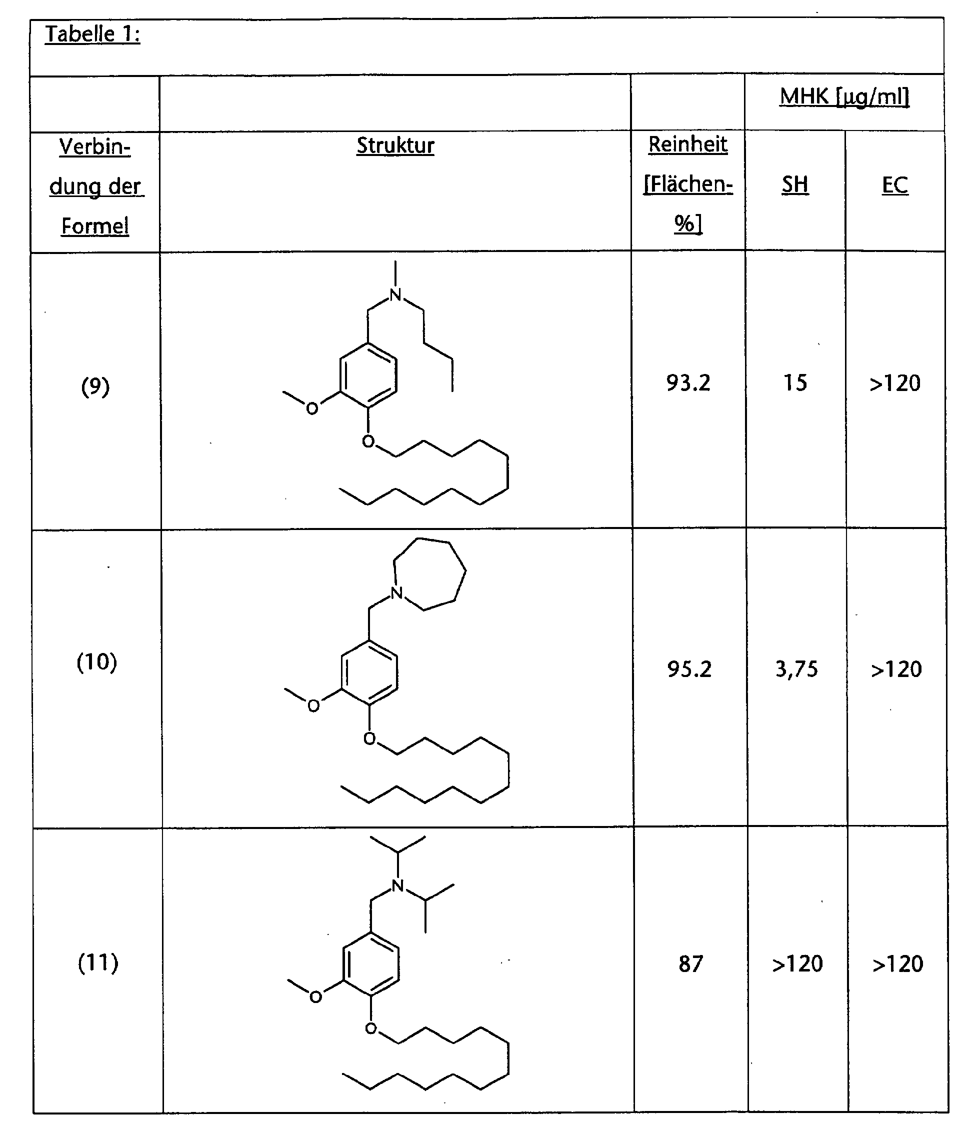

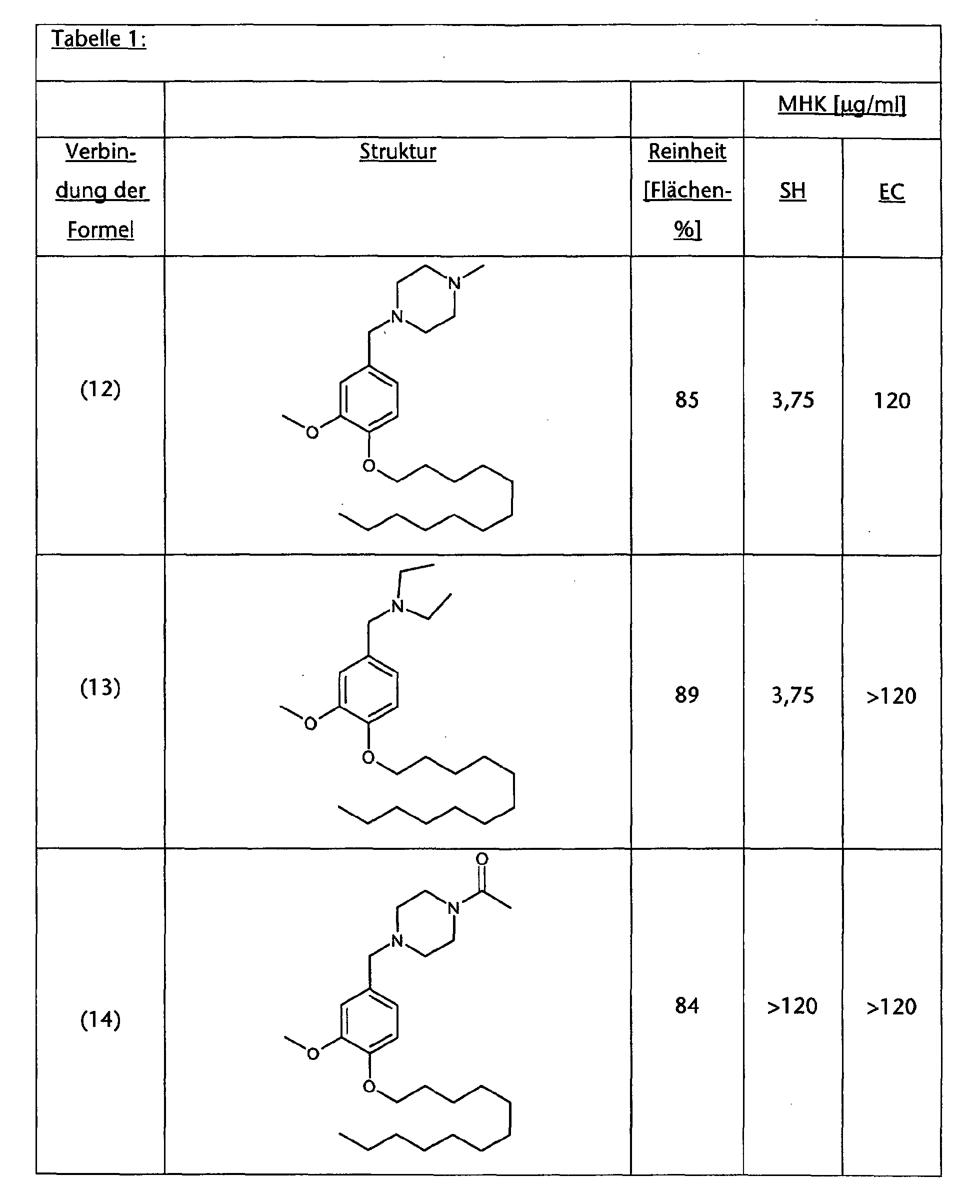

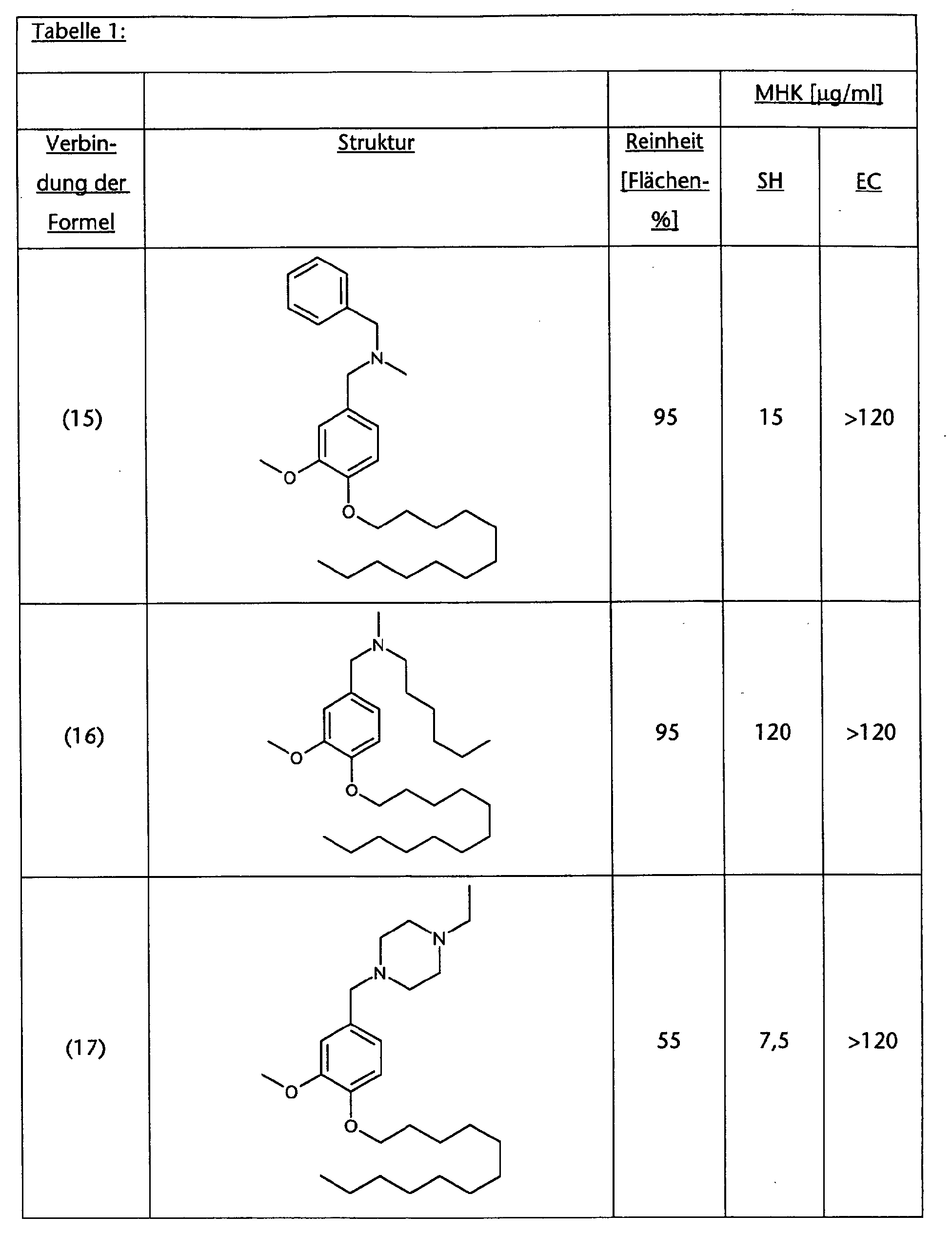

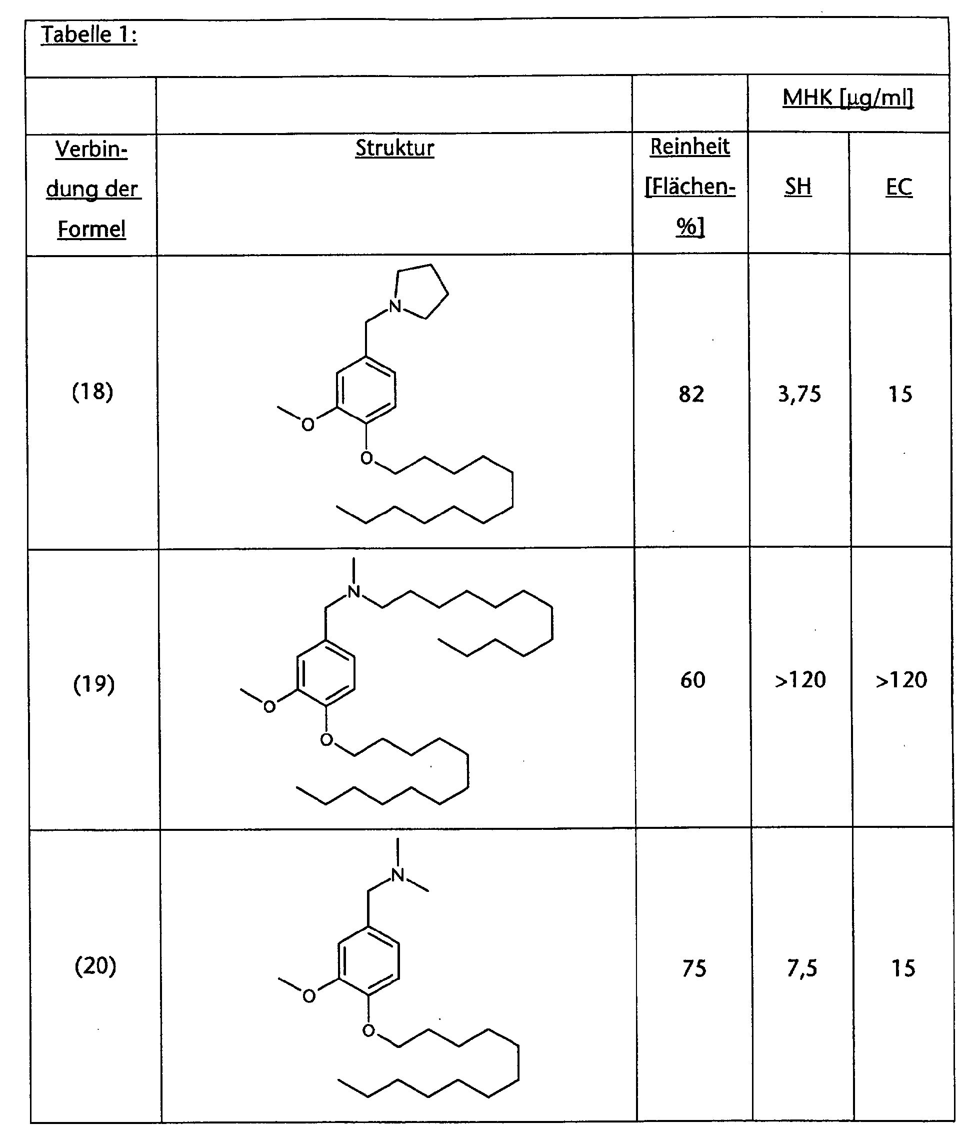

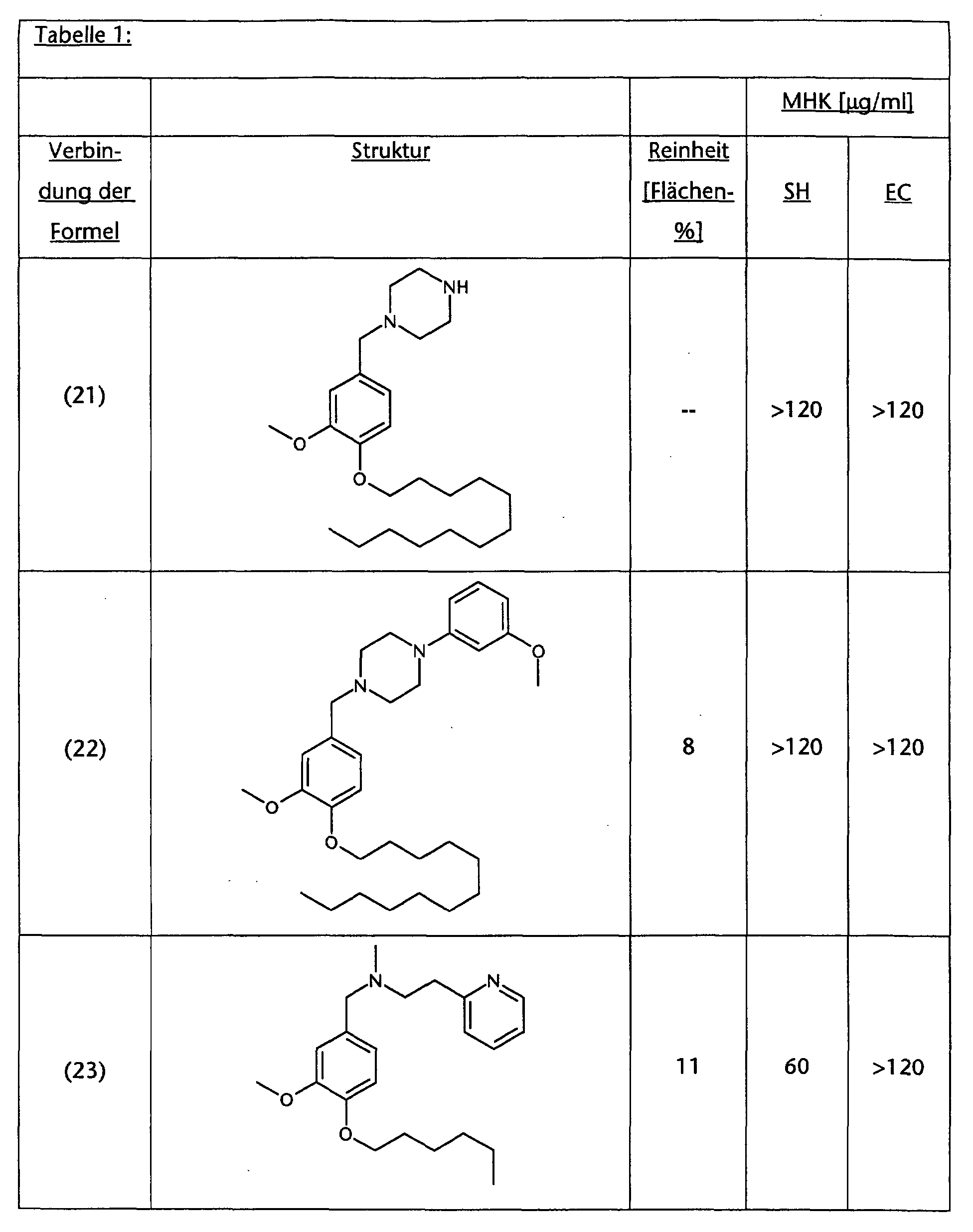

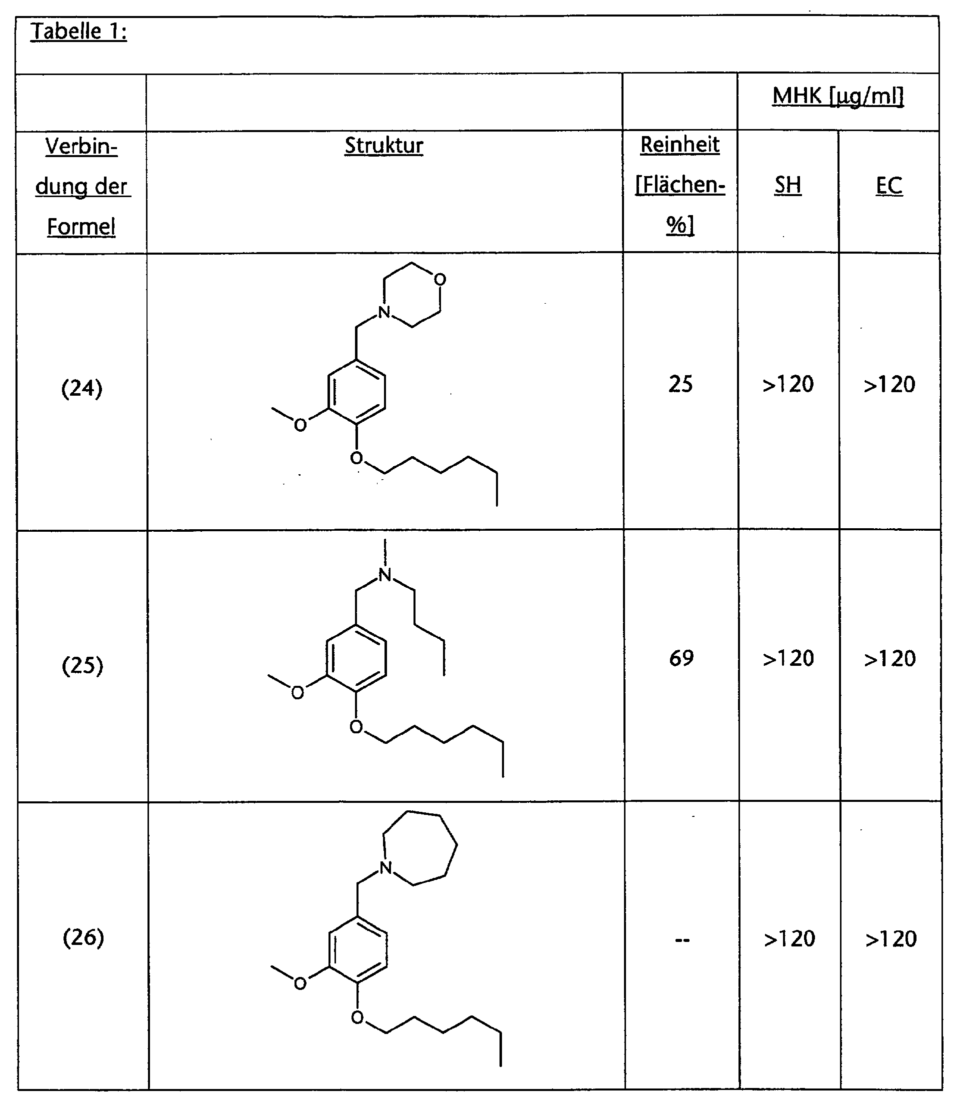

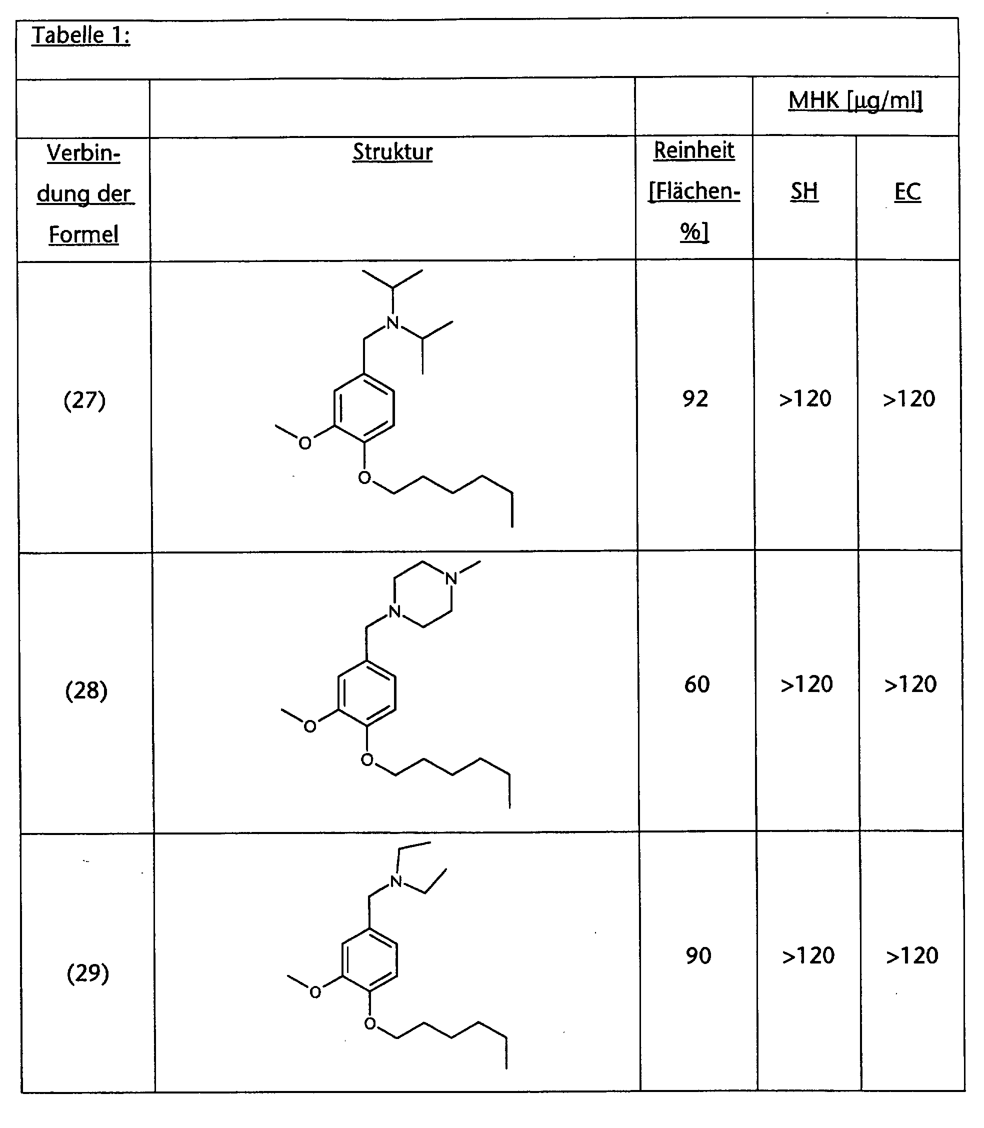

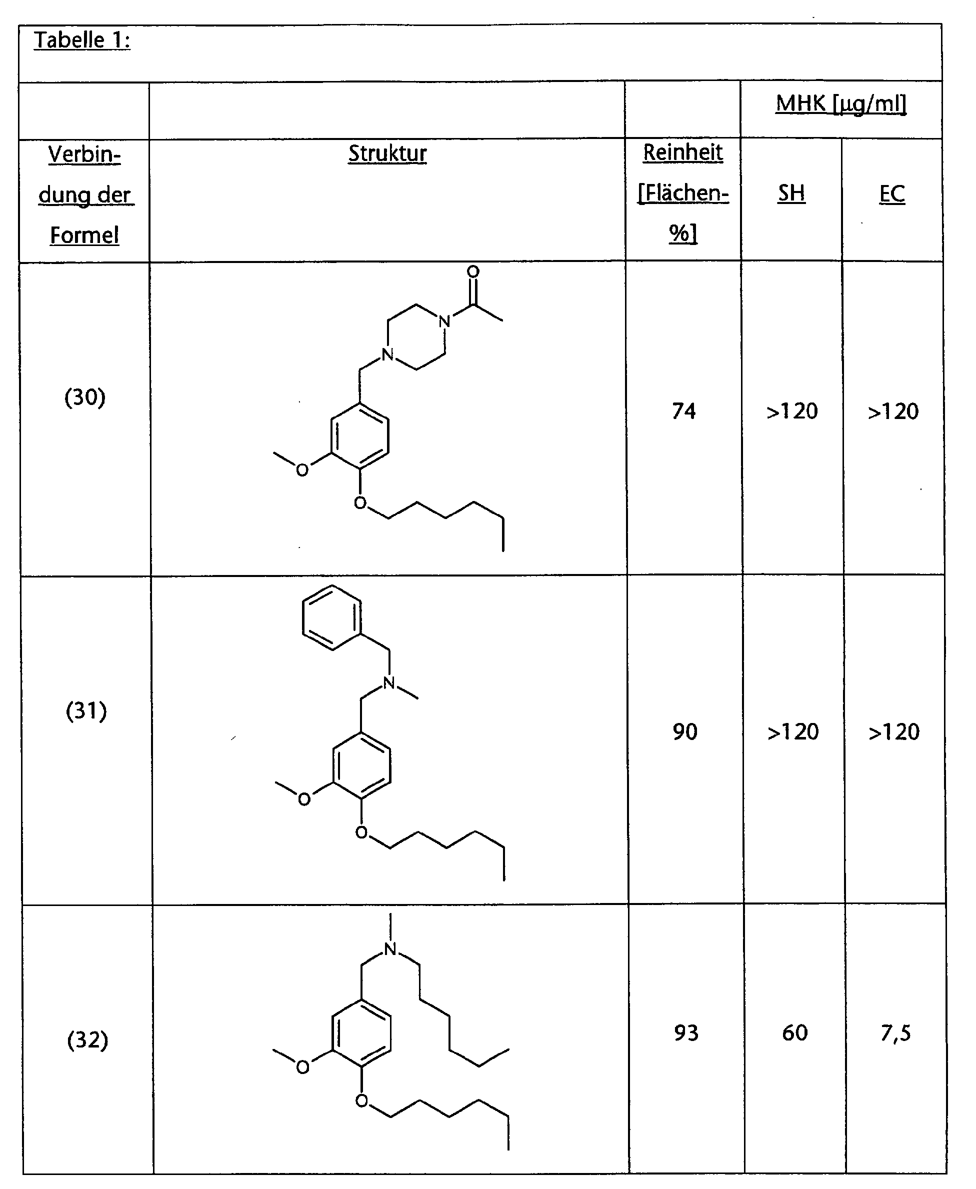

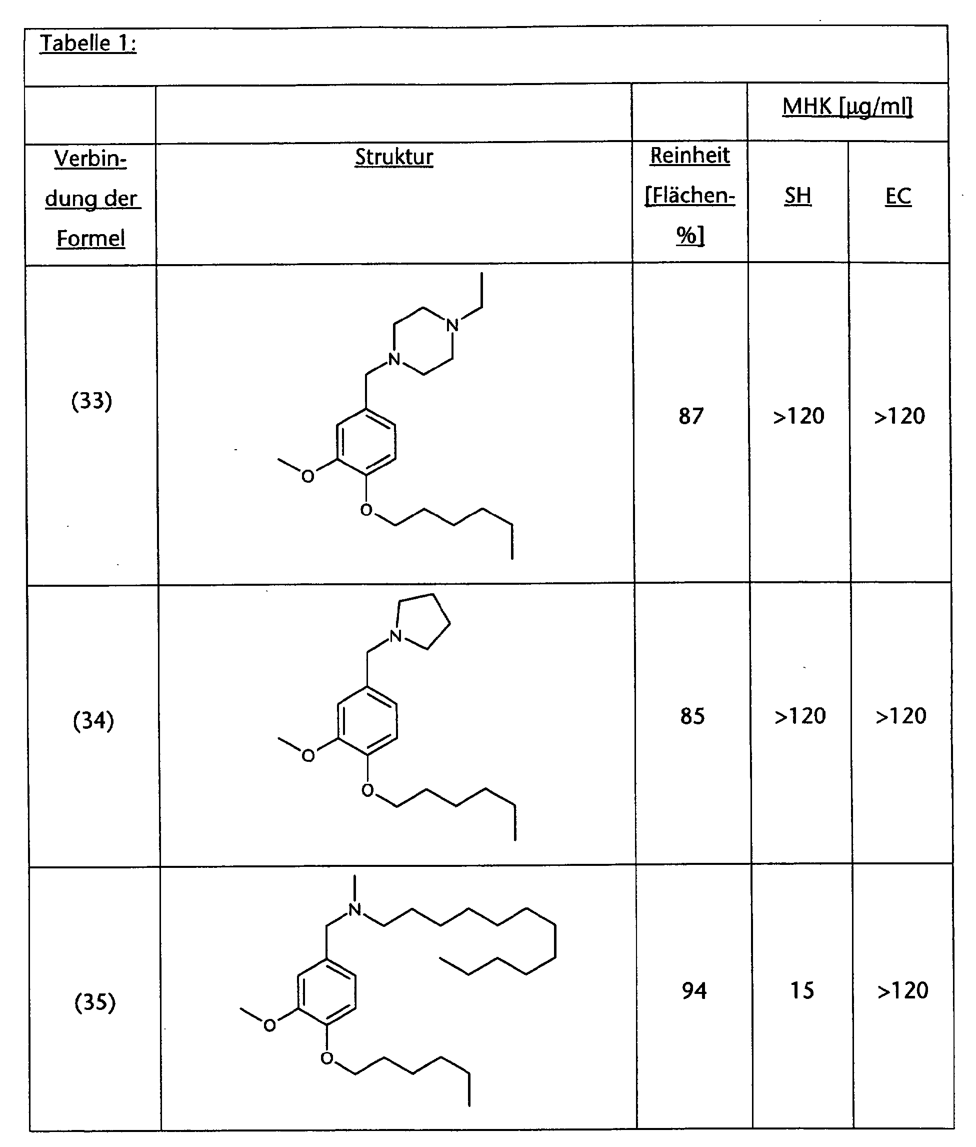

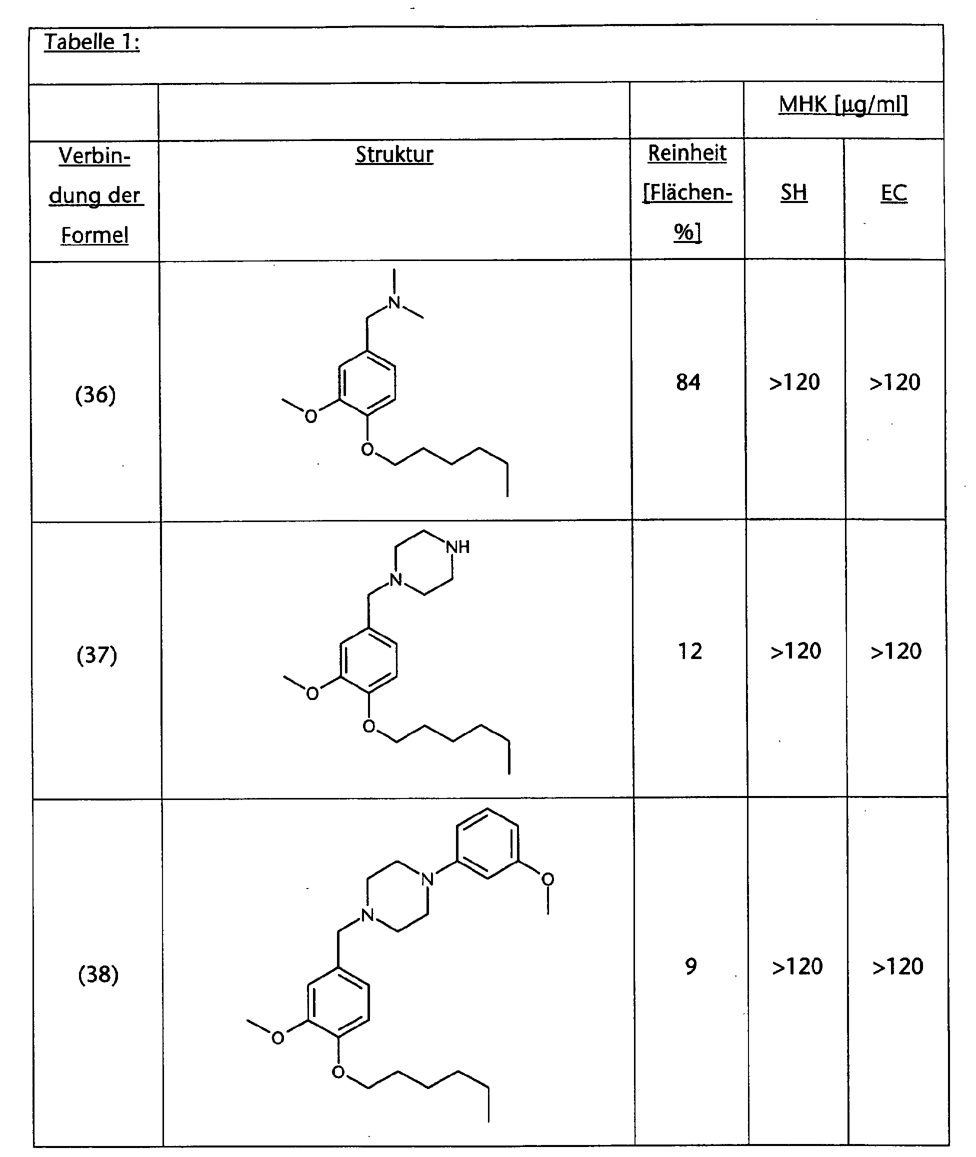

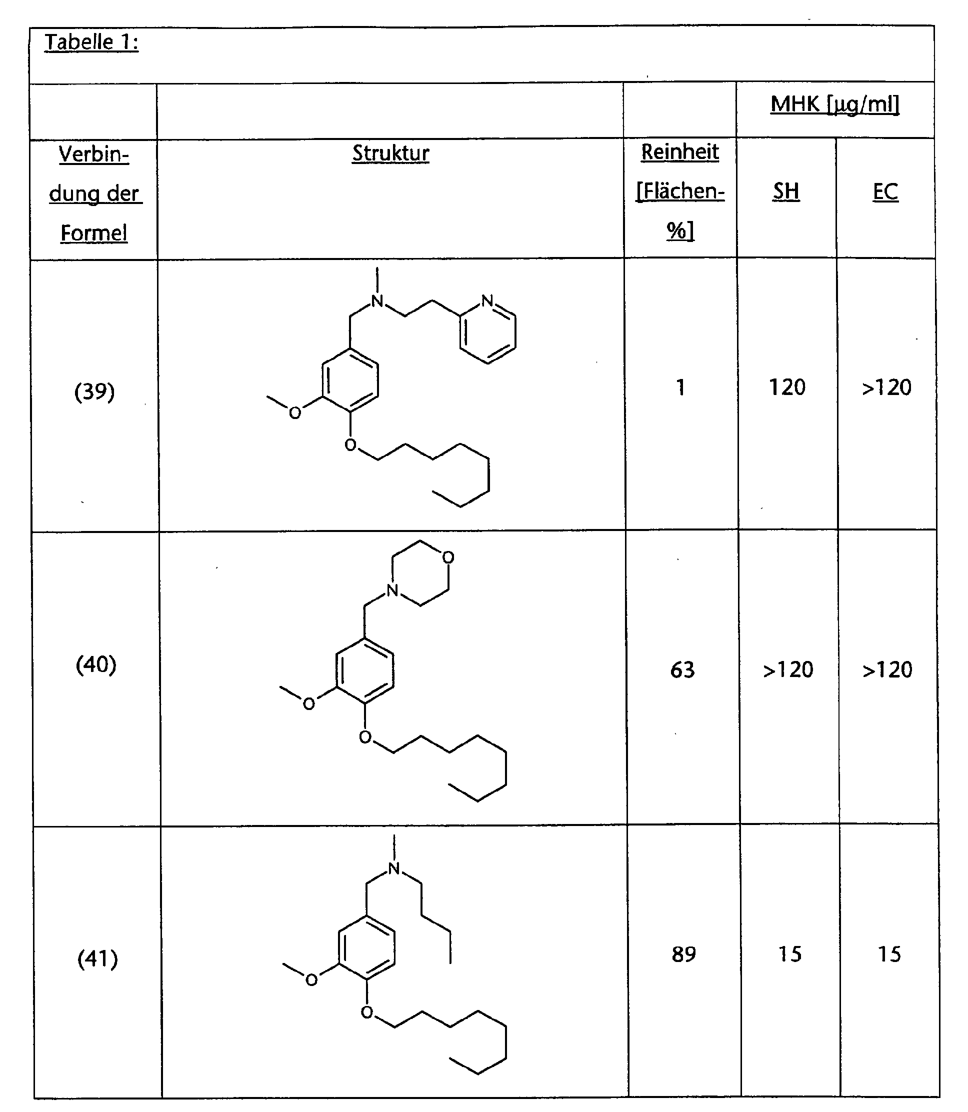

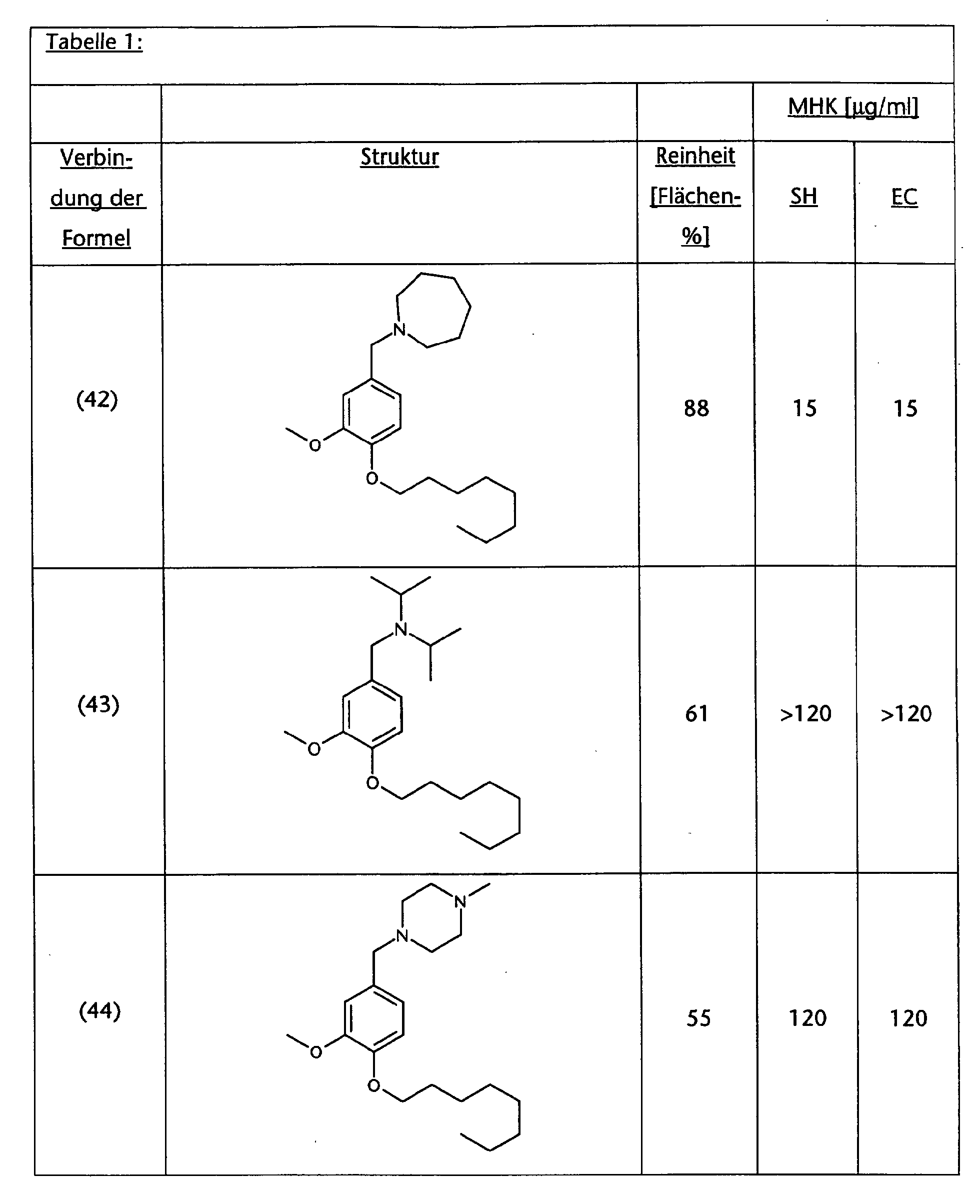

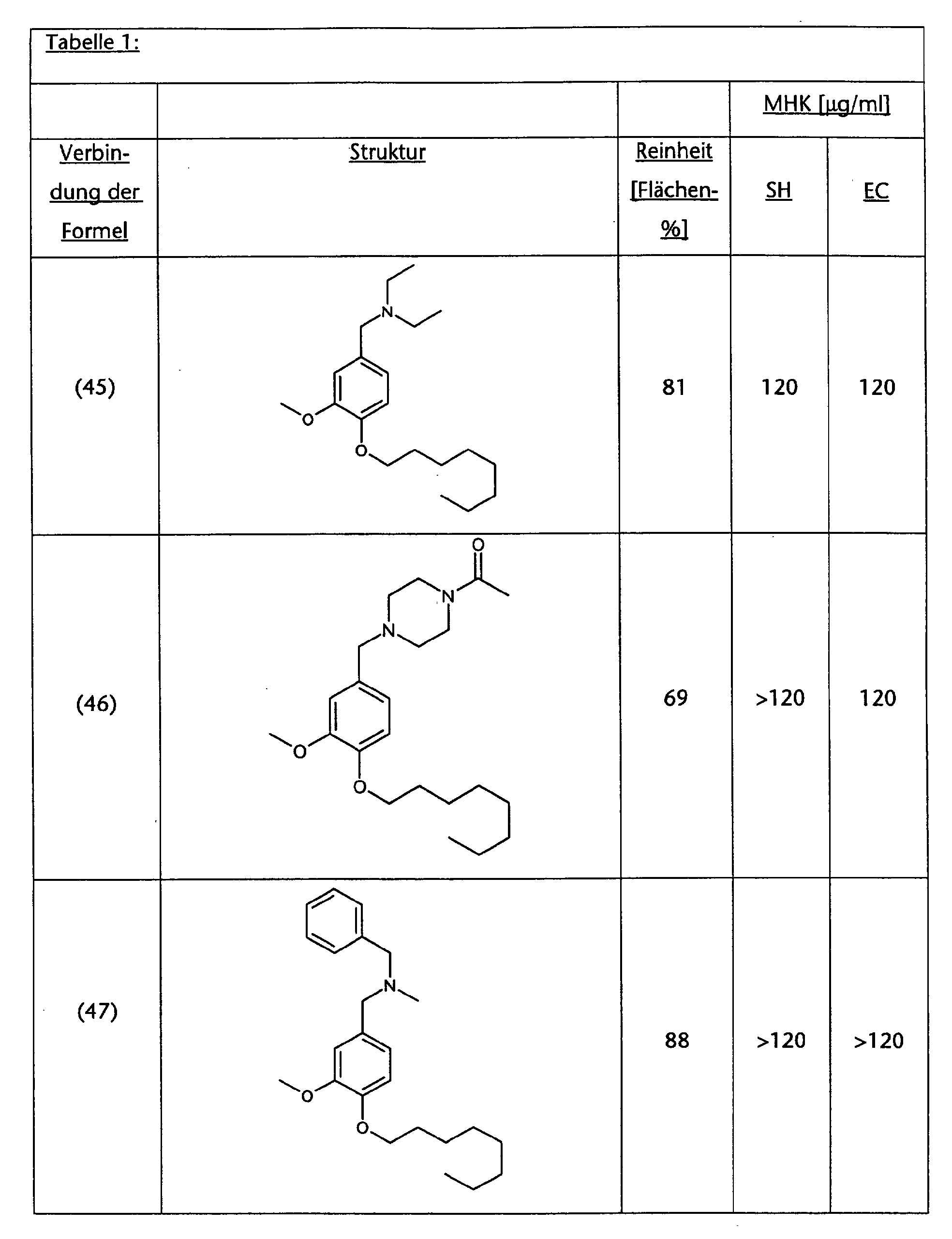

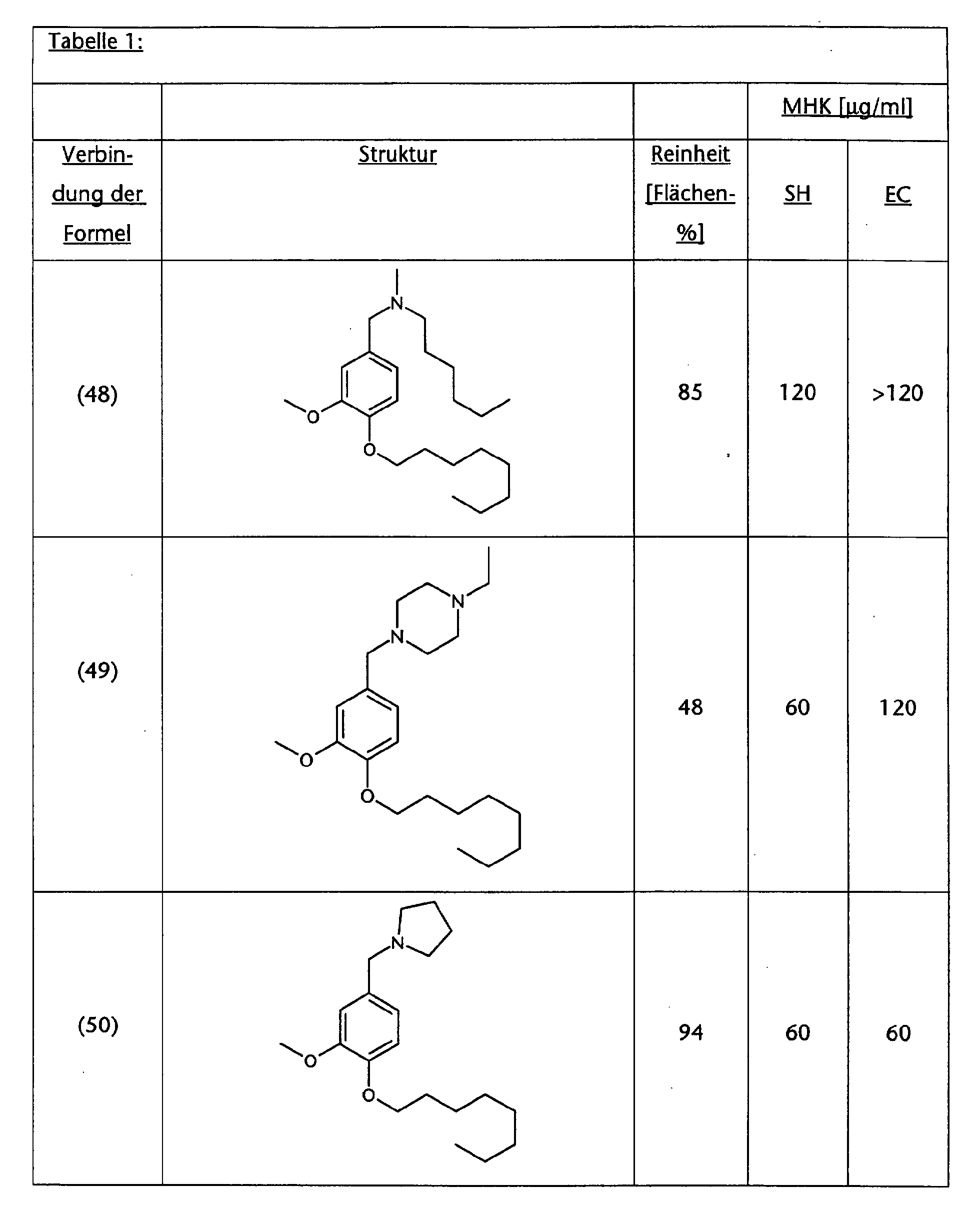

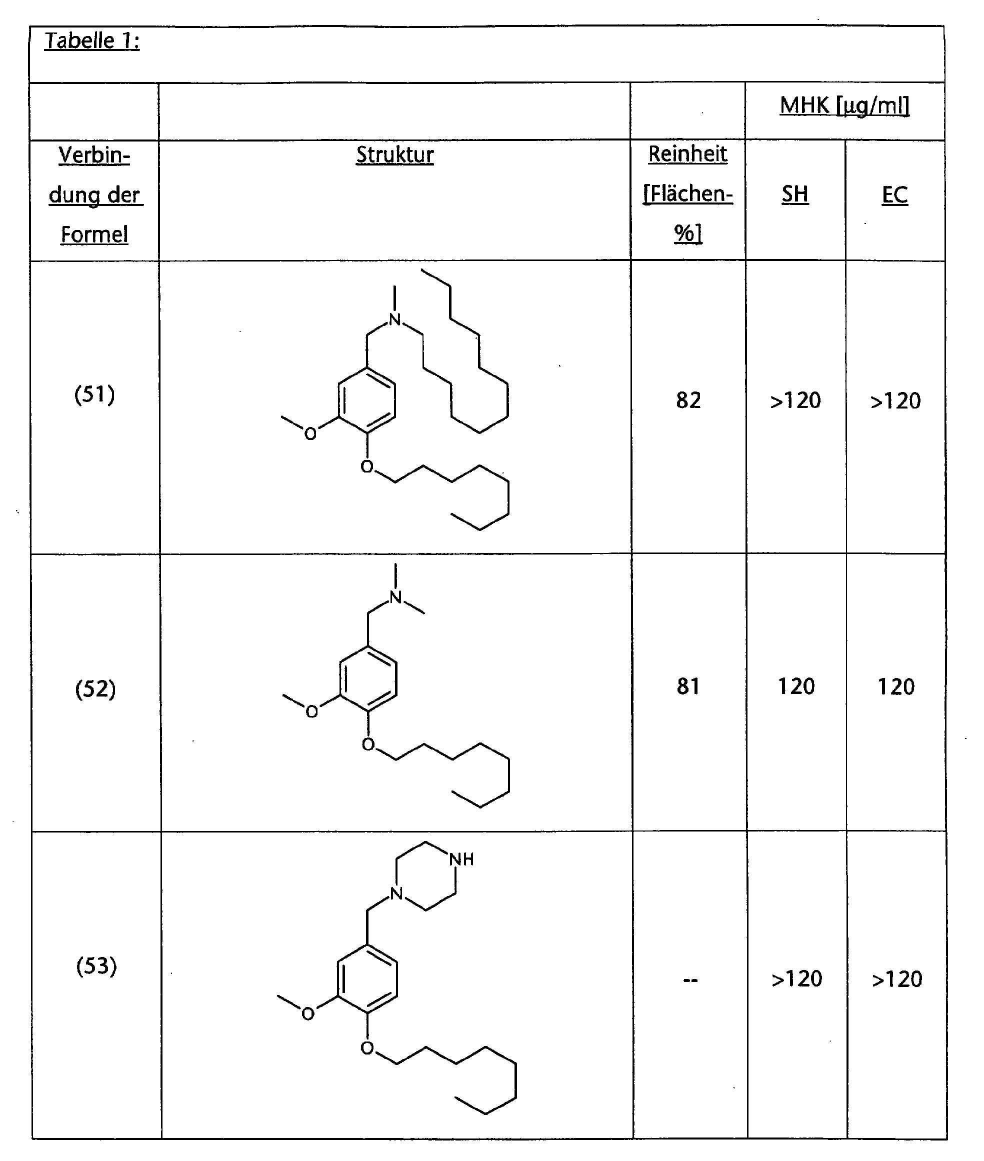

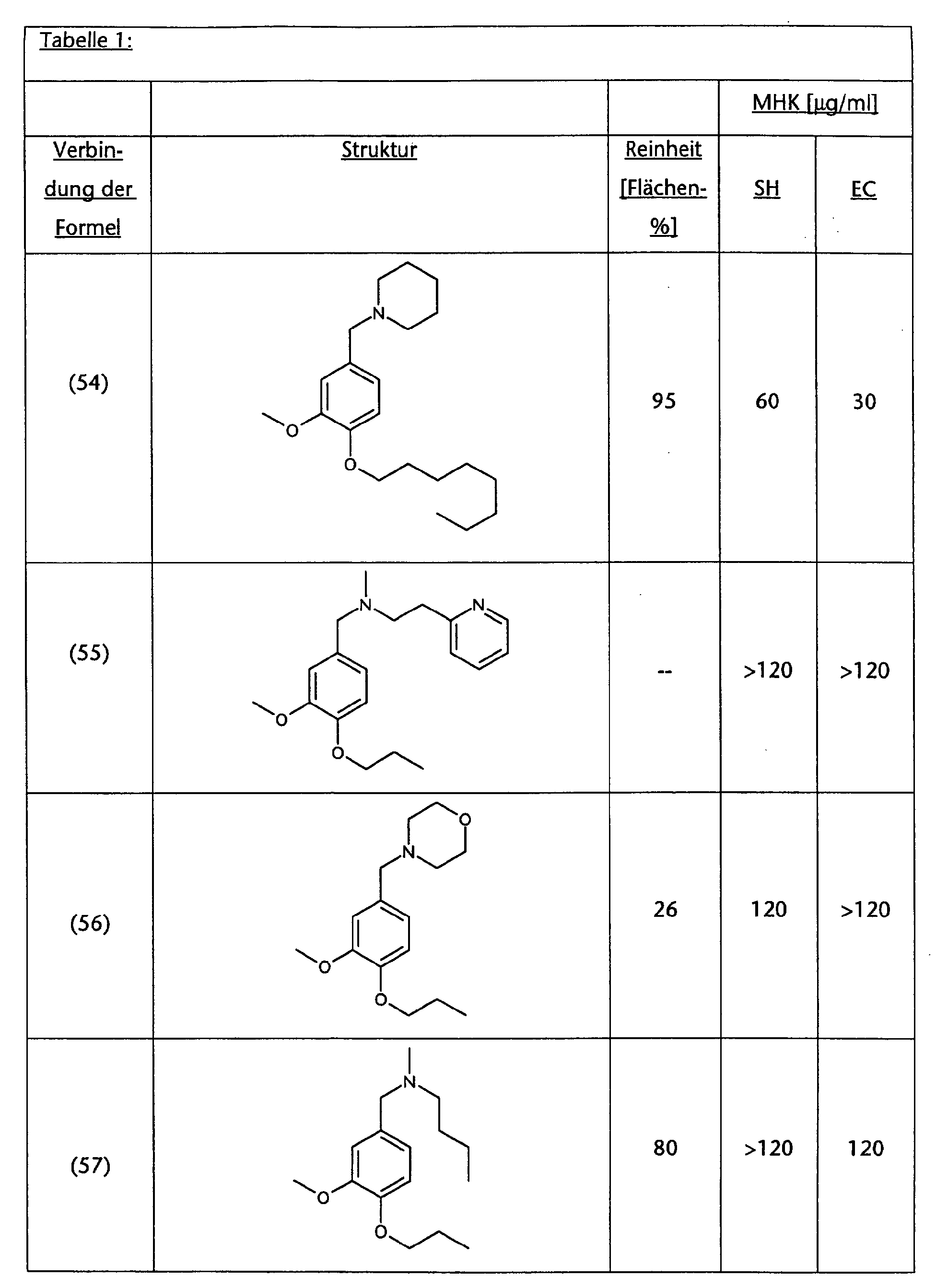

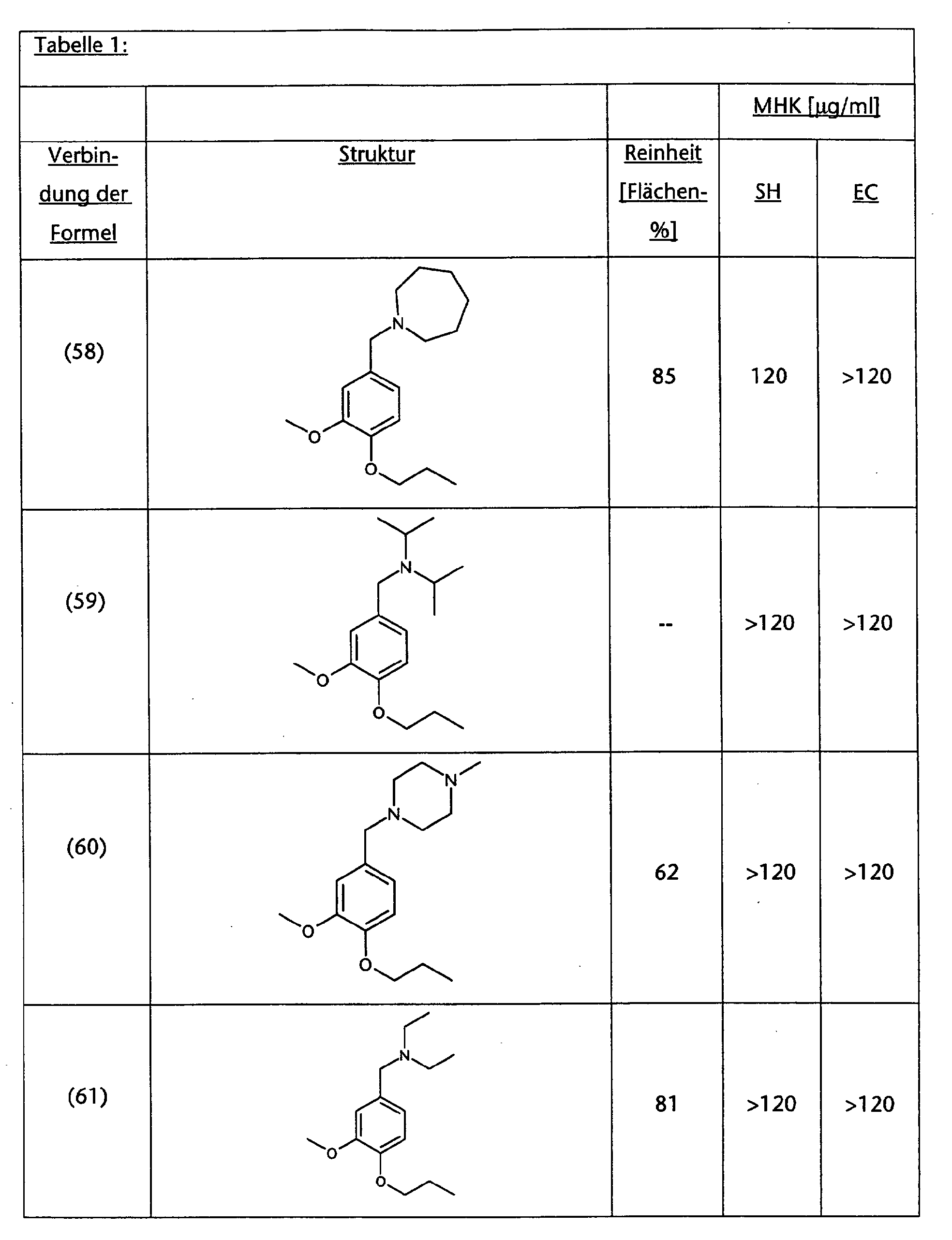

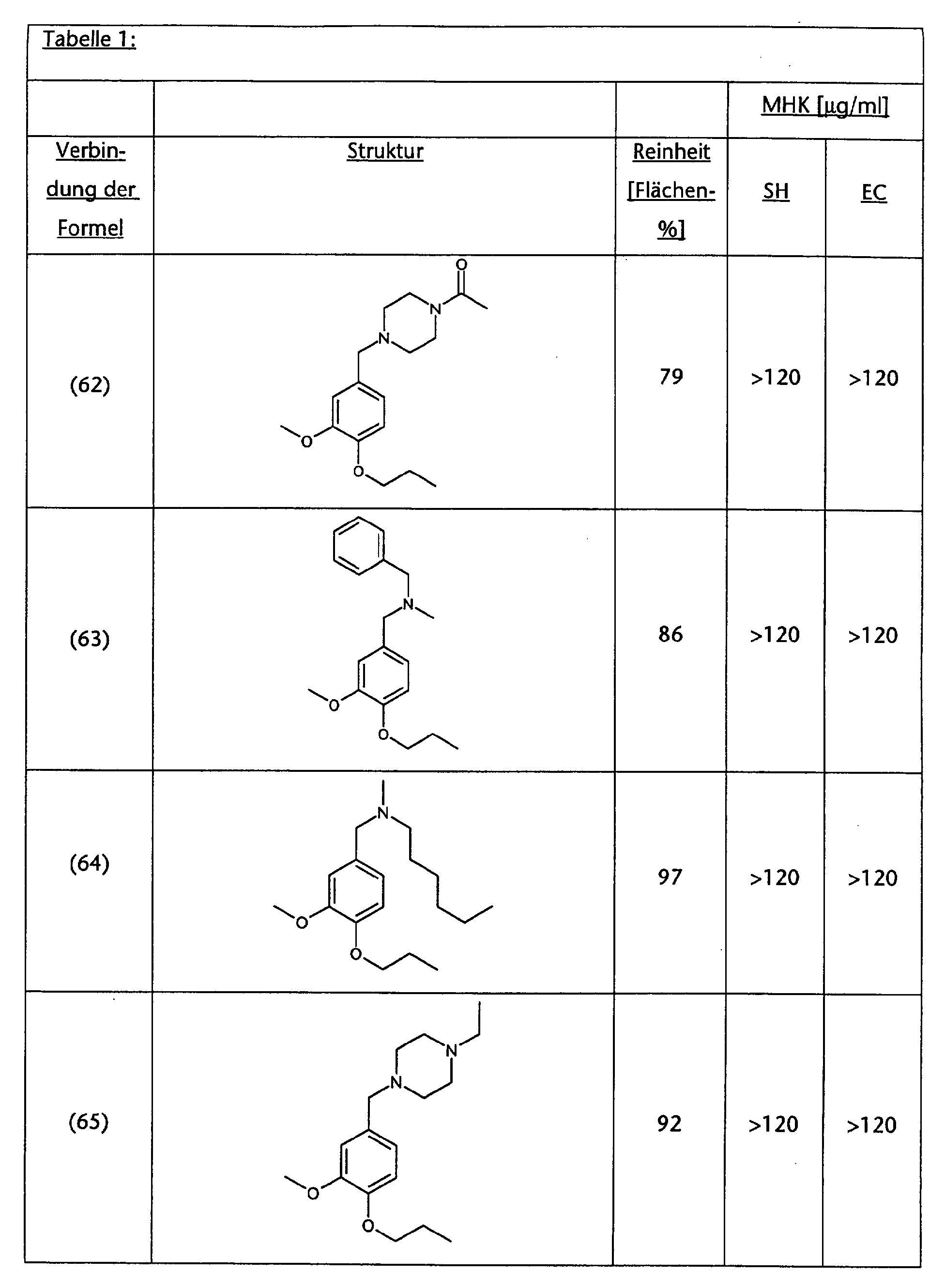

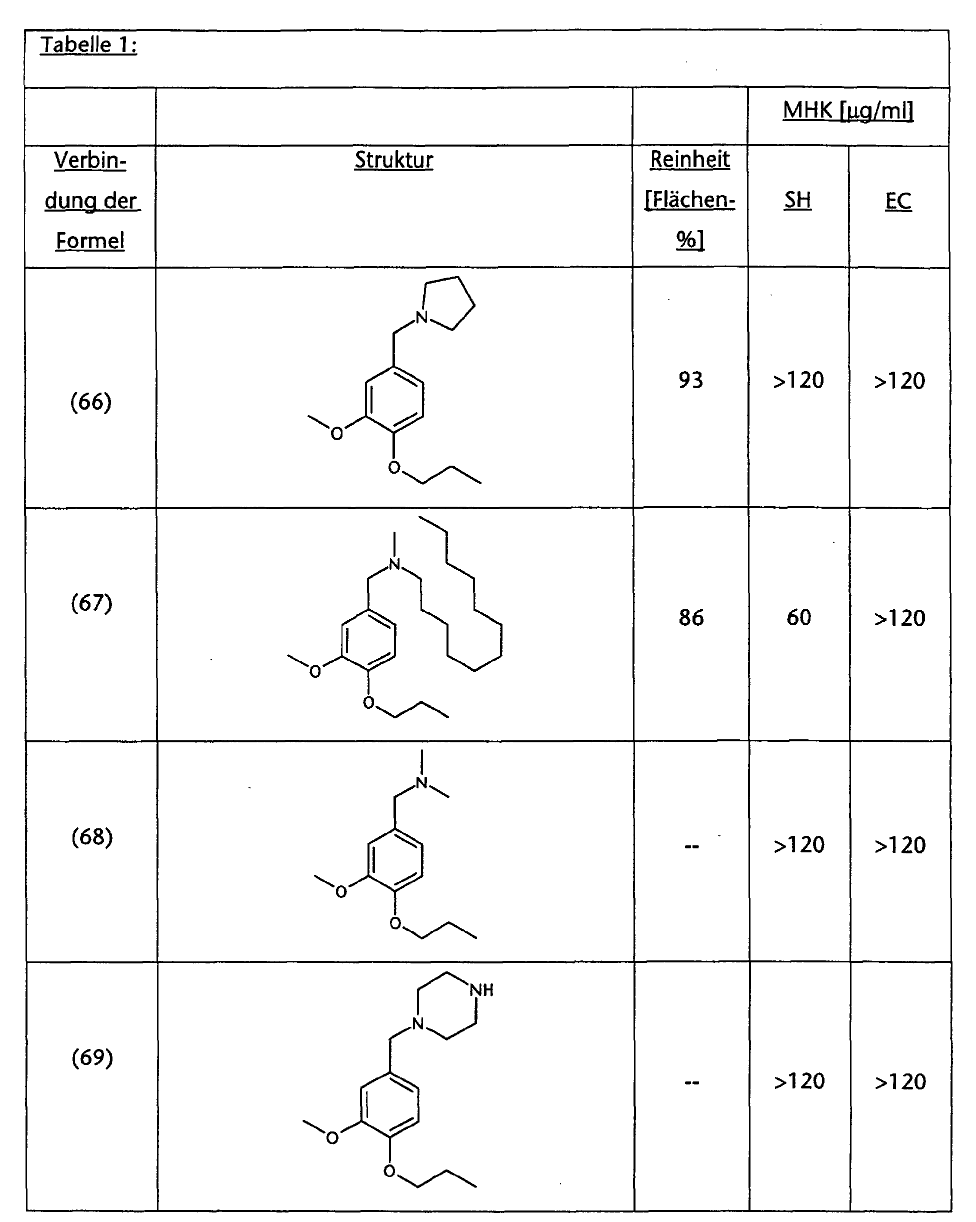

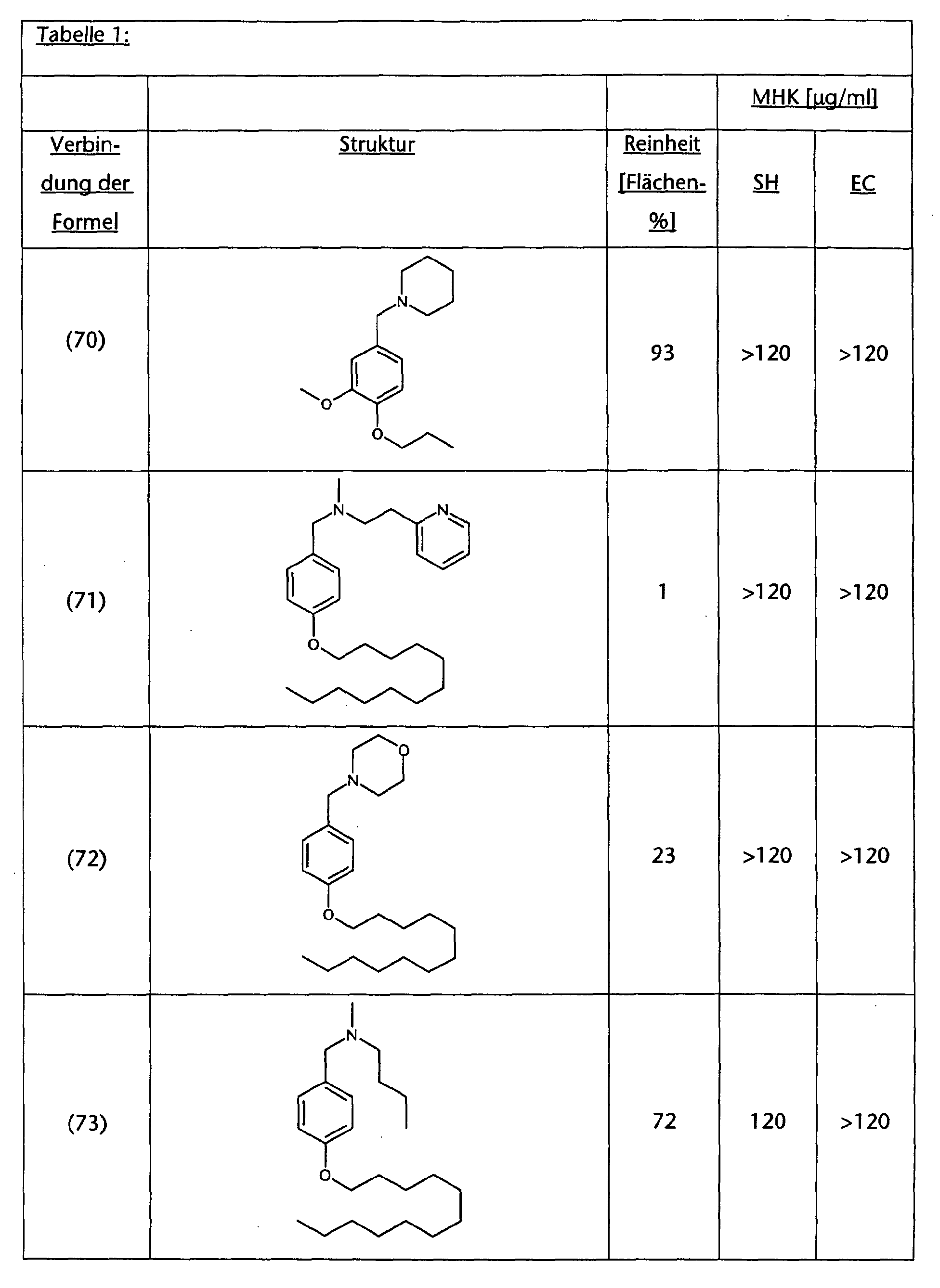

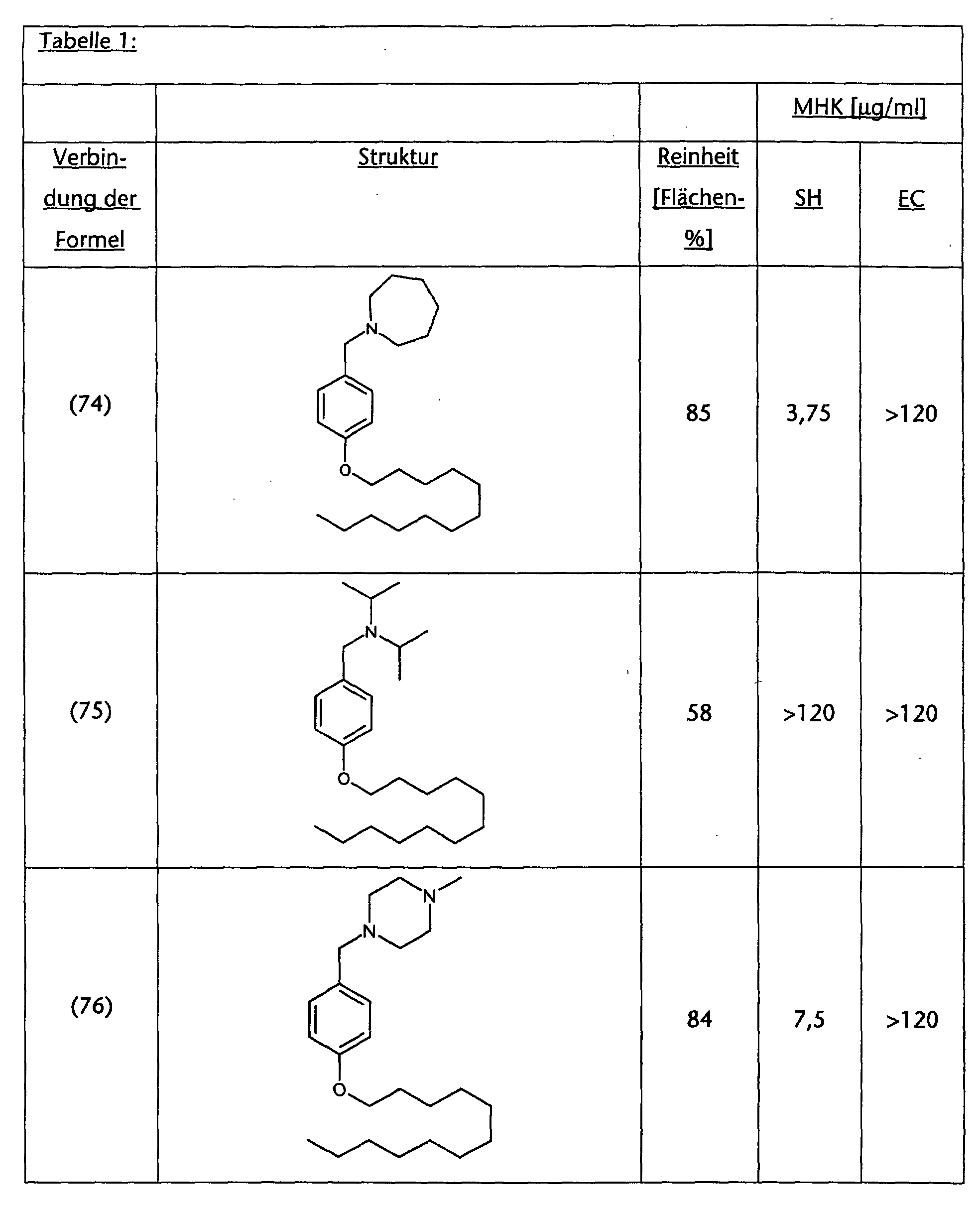

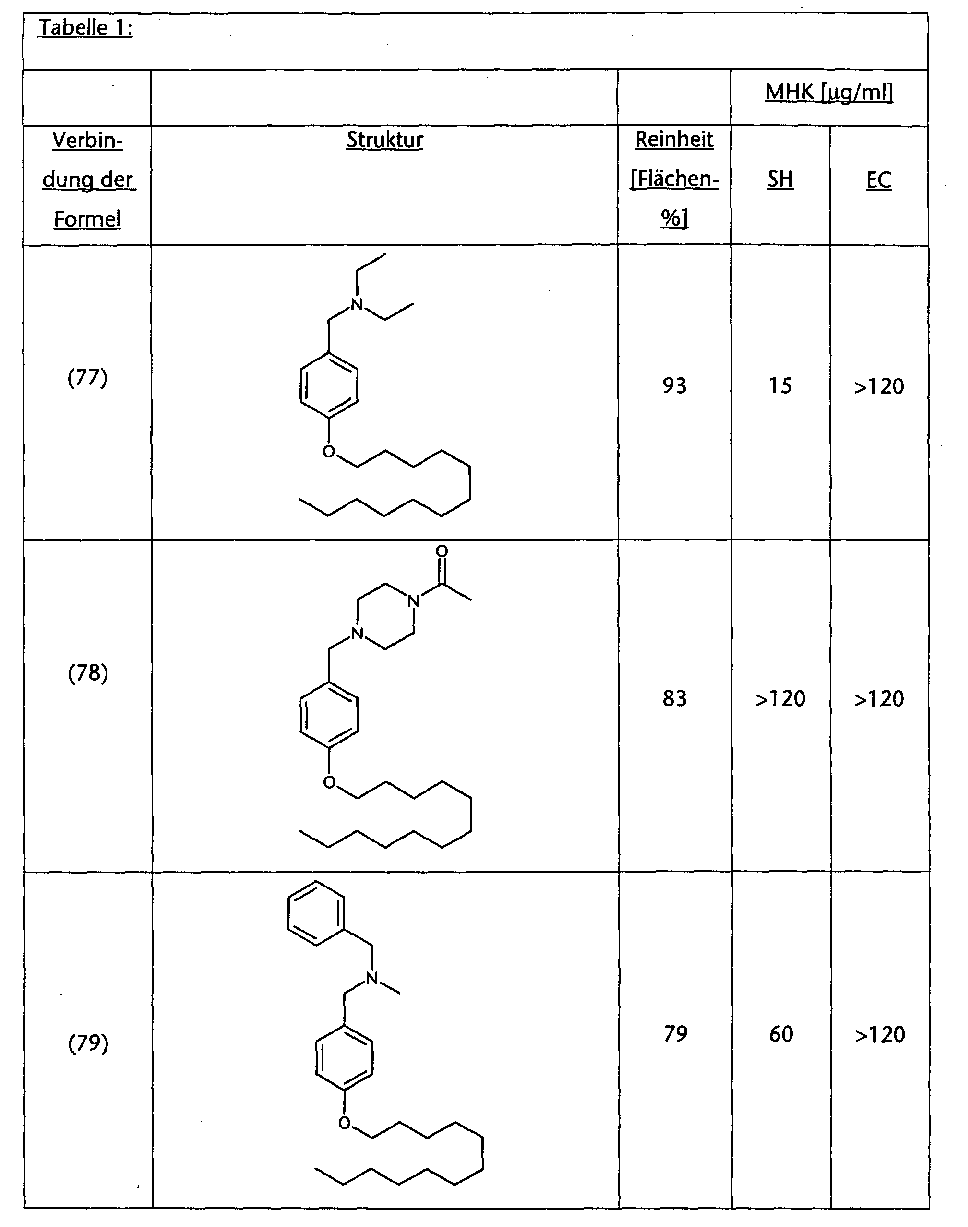

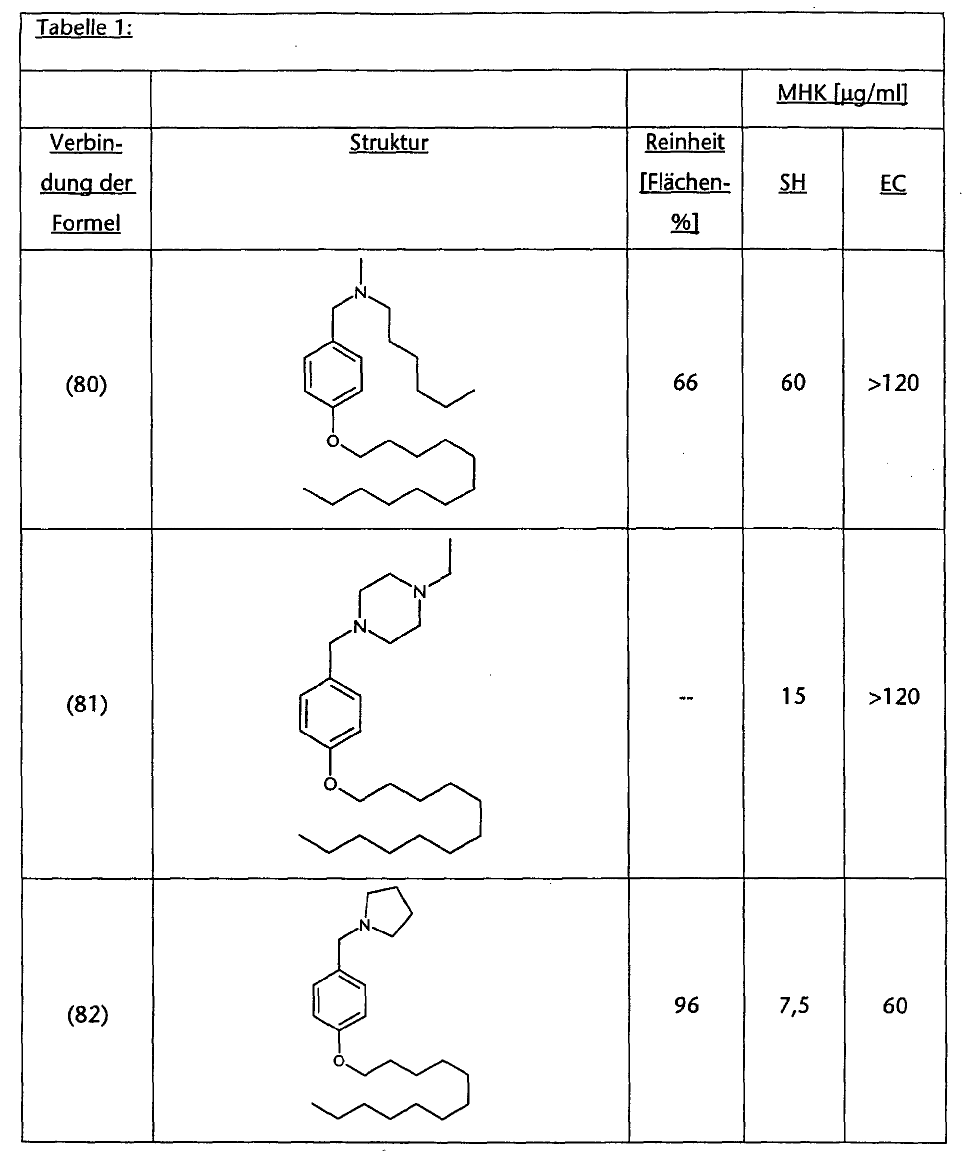

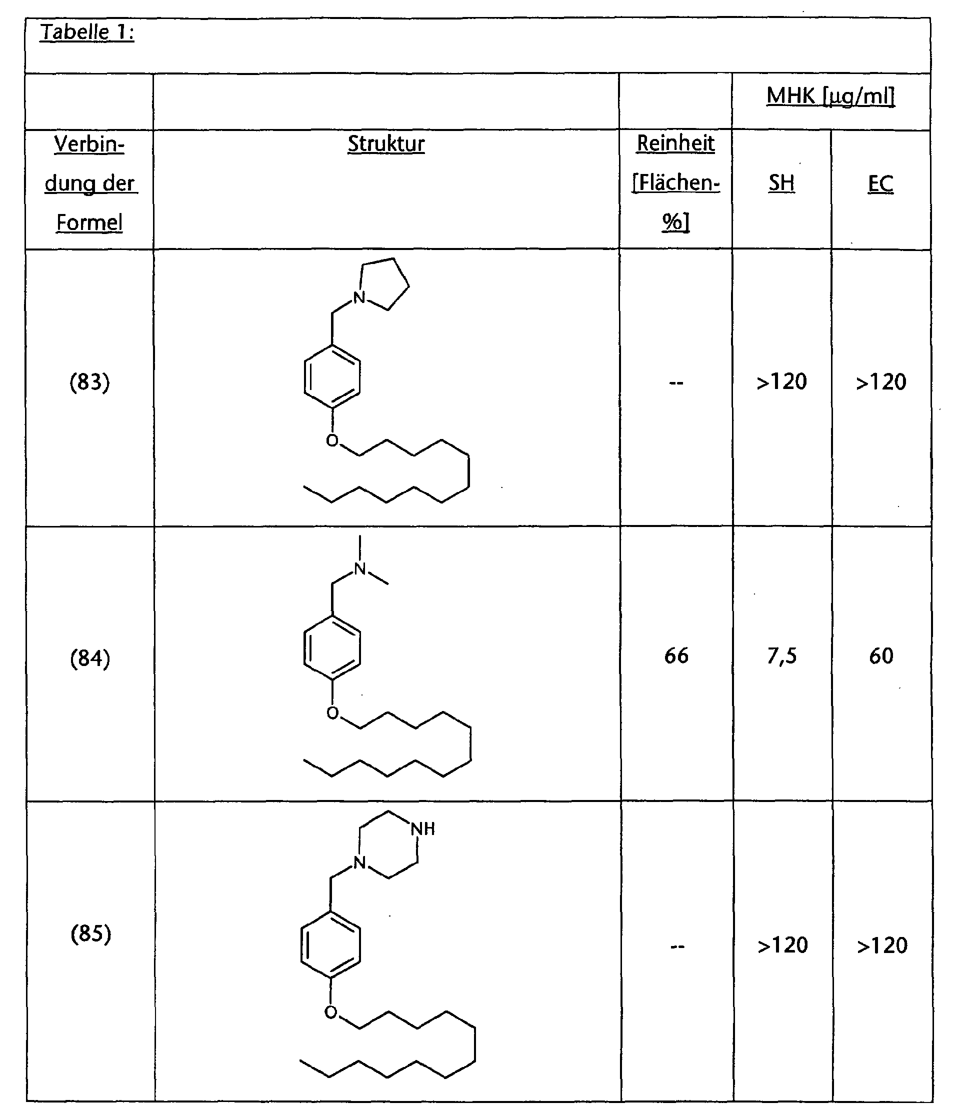

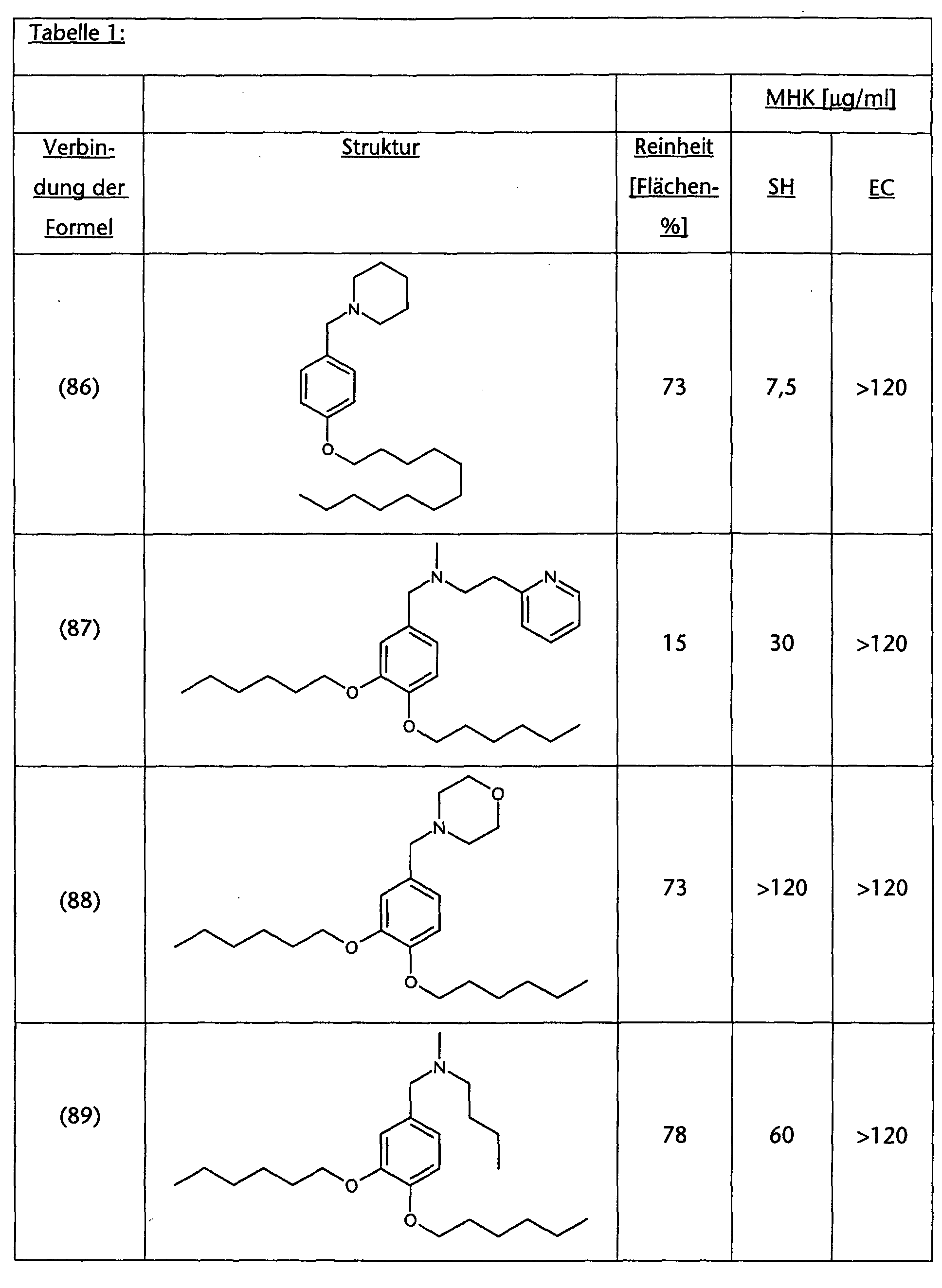

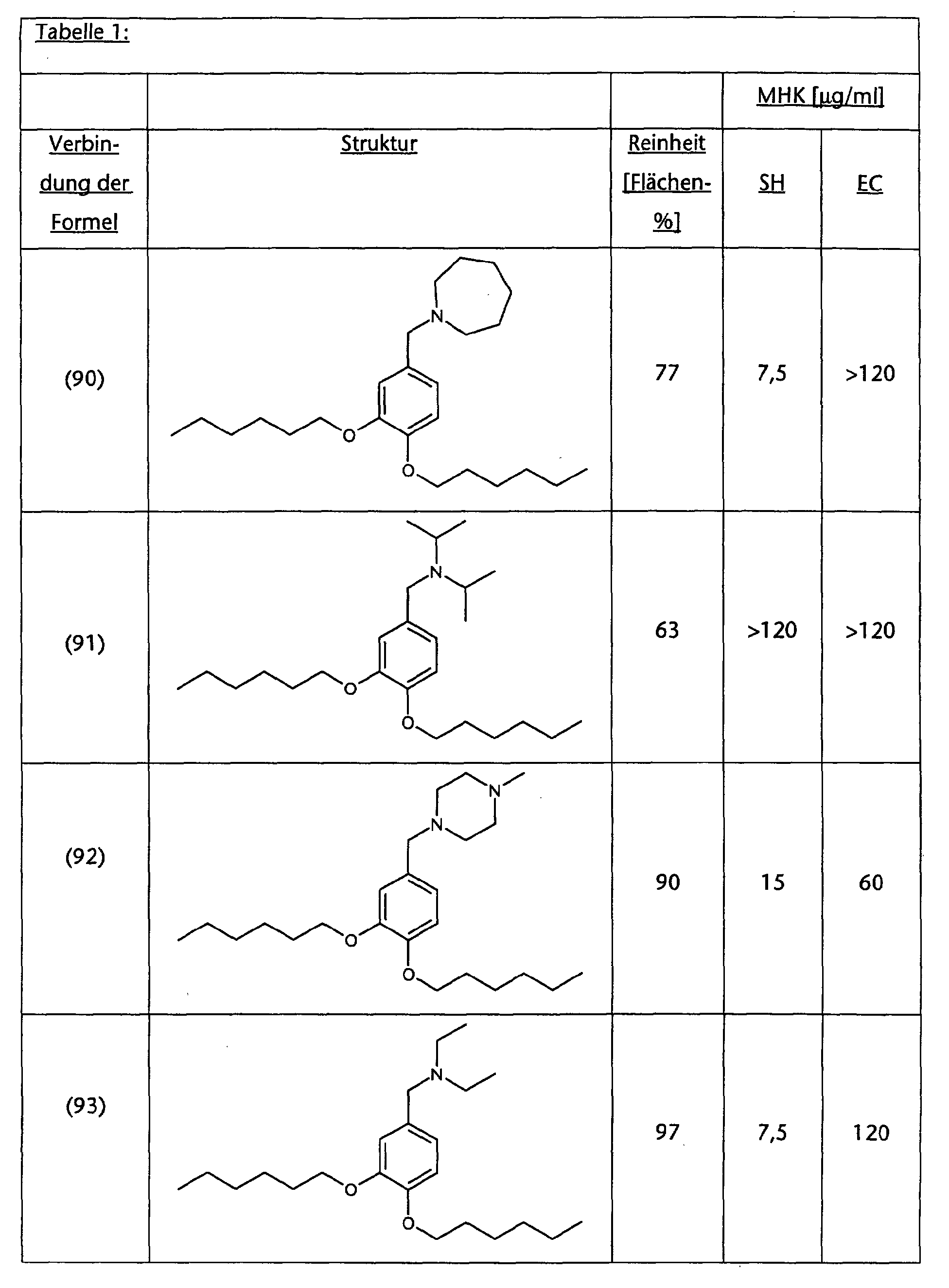

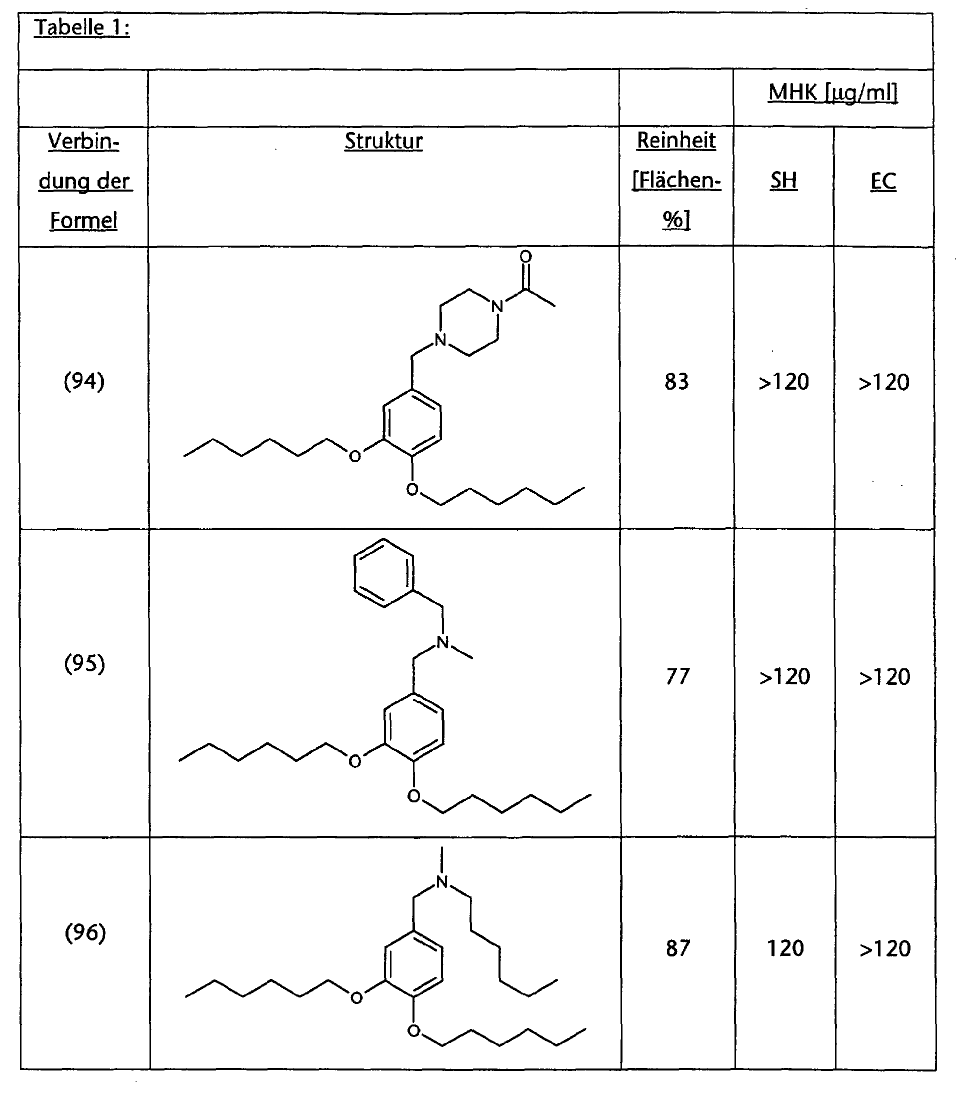

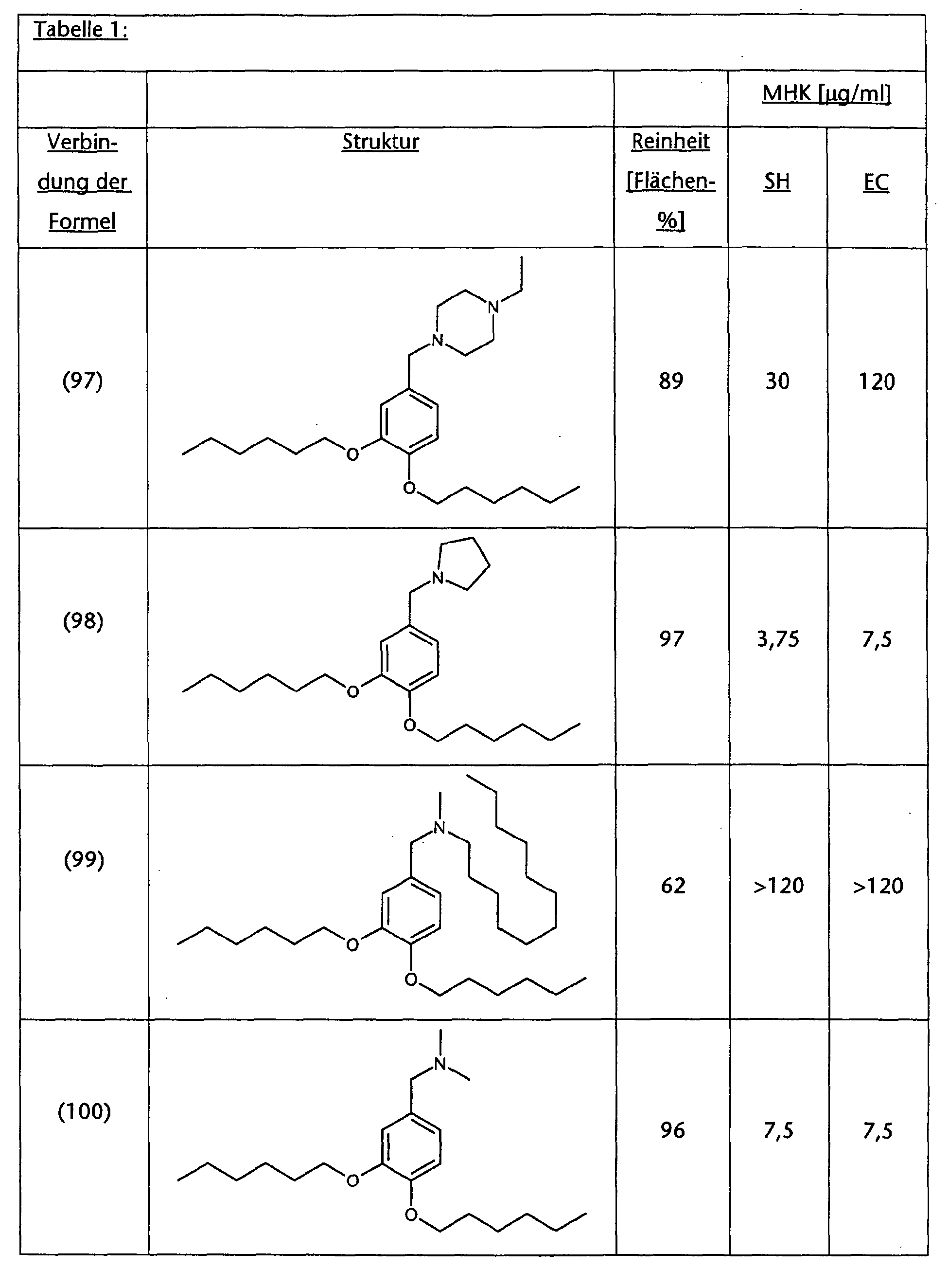

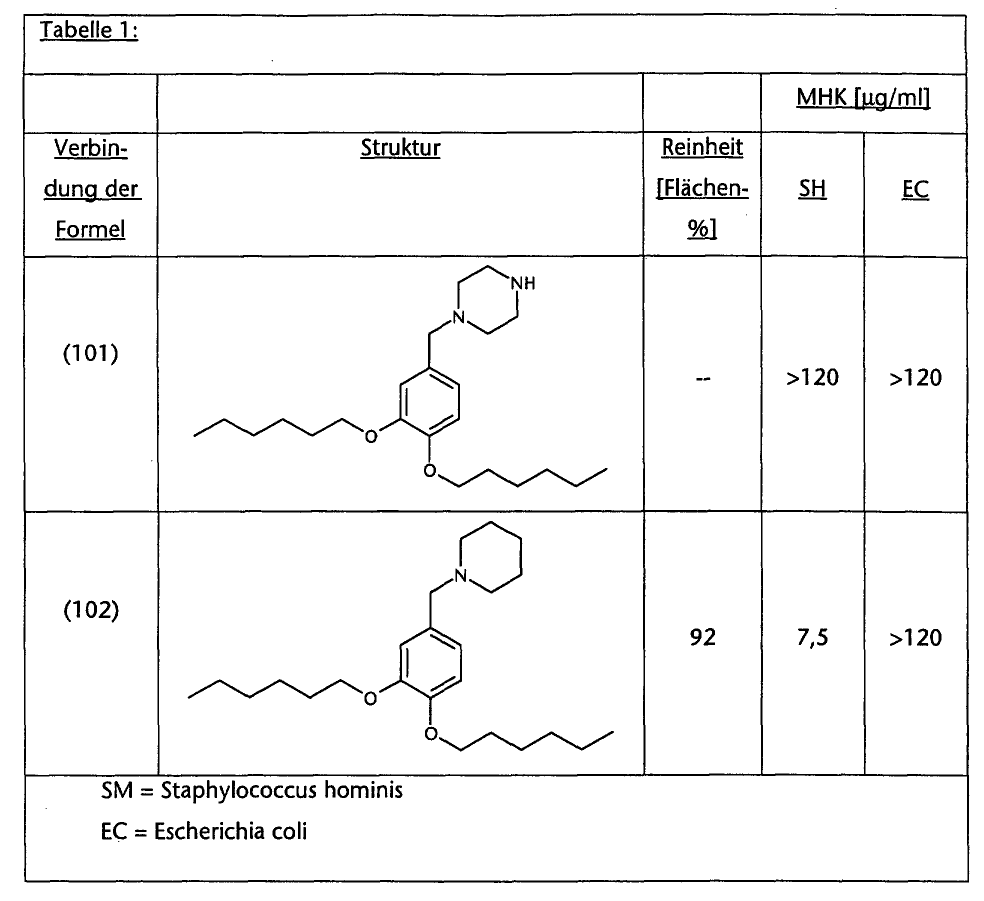

### Bestimmung der minimalen Hemmkonzentration (MHK-Wert) in Mikrotiterplatten

### Nährmedium:

Casein-Sojamehl-Pepton-Bouillon zur Herstellung der Vorkulturen der Testbakterien und Hefe.

### Verwendete Testkeime:

Staphylococcus hominis DSM 20328
Escherichia coli NCTC 8196

### Durchführung:

Die Testsubstanzen werden in Dimethylsulfoxid (DMSO) vorgelöst und in einer Verdünnungsreihe von 1:2 getestet.

Die Bakterien werden über Nacht in CASO-Bouillon angezüchtet.

Alle Testkeime werden mit 0.85% Kochsalzlösung auf eine Keimzahl von 1-5*10⁶ KBE/ml eingestellt.

Die Testsubstanzen werden à 8µl pro well in Mikrotiterplatten vorpipettiert.

Vorverdünnte Testkeimsuspensionen werden 1:100 in CASO-Bouillon verdünnt und à 192µl pro well den Testsubstanzen zugegeben.

Die Testansätze werden 48 Stunden bei 37°C inkubiert.

Nach Inkubation wird das Wachstum anhand der Trübung der Testansätze (Optische Dichte) bei 620 nm in einem Mikroplate-Reader bestimmt.

Als minimale Hemmkonzentration (MHK-Wert) wird diejenige Substanzkonzentration angegeben, bei der (verglichen mit der Wachstumskontrolle) eine deutliche Wachstumshemmung (≤ 20% Wachstum) der Testkeime festzustellen ist.

Pro Testkeim und Substanzkonzentration wird eine Mikrotiterplatte angesetzt.

Alle Substanzen werden im Doppel geprüft.

Die erfindungsgemässen Alkyloxybenzylamine werden nach gängigen Synthesemethoden hergestellt. Dazu wird in einem ersten Reaktionsschritt ein Alkyloxyhydroxybenzaldehyd, ein Hydroxybenzaldehyd oder ein Dihydroxybenzaldehyd mit dem entsprechenden Alkylhalogenid in einem geeignetem Lösungsmittel, wie z.B. Toluol, Xylol, DMF oder THF bei einer Temperatur zwischen 40 und 130°C innerhalb 1 bis 24 Stunden unter Verwendung einer Hilfsbase,wie z.B. Natriumalkoholate (--methylat, -ethylat, -tert.butylat), Soda, Pottasche oder Natriumhydroxyd zum entsprechenden Mono- bzw. Bisalkyloxybenzaldehyd alkyliert. Danach wird der entsprechende Mono- bzw. Bisalkyloxybenzaldehyd in einem geeigneten Lösungsmittel wie z.B. THF, Dioxan, Toluol oder in einem Überschuss eines sekundären Amins ohne Lösungsmittel mittels eines wasserentziehenden Agens, wie z.B. Trimethylorthoformiat, Molsieb oder durch azeotrope Abdestillation von Wasser mit einem sekundären Amin kondensiert. Durch gleichzeitige Zugabe eines Reduktionsmittels wie z.B. Natriumcyanoborhydrid, Natriumborhydrid, Lithiumaluminiumhydrid oder Ameisensäure werden bei einer Temperatur zwischen -10 - 120 °C innerhalb von 0,5 - 24 h direkt die entsprechenden Mono- bzw. Bisalkyloxybenzylamine erhalten.

Der gesamte Reaktionsablauf lässt sich mit folgendem Schema darstellen:

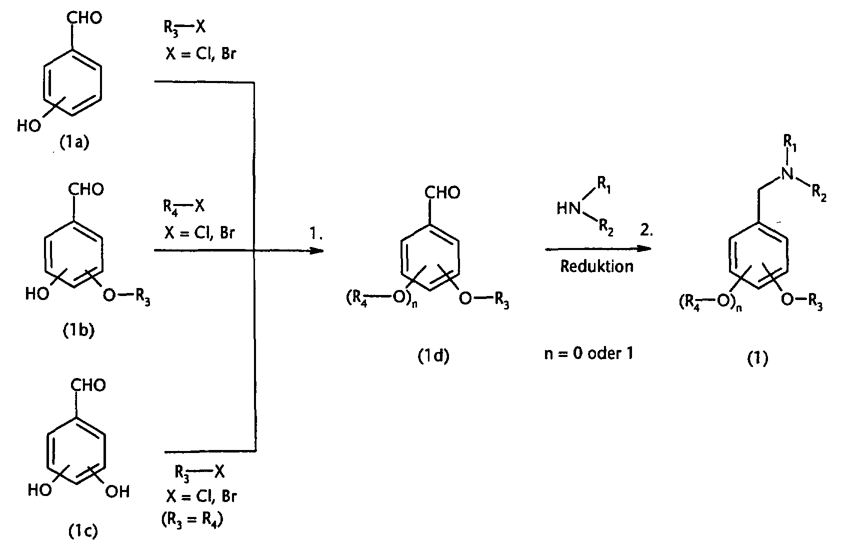

Die erfindungsgemäss eingesetzten Alkoxybenzylamine zeigen ausgeprägte antimikrobielle Wirkung, insbesondere gegen pathogene grampositive und gramnegative Bakterien sowie gegen Bakterien der Hautflora, ausserdem gegen Hefen und Schimmelpilze.

Sie eignen sich daher insbesondere zur Desinfektion, Desodorierung, sowie der allgemeinen und antimikrobiellen Behandlung der Haut und Schleimhäute sowie Hautanhangsgebilde (Haare), ganz besonders zur Hände- und Wunddesinfektion.

Sie sind daher geeignet als antimikrobielle Wirksubstanzen und Konservierungsmittel in Körperpflegemitteln, wie z.B. Shampoos, Badezusätzen, Haarpflegemitteln, flüssigen und festen Seifen (auf Basis synthetischer Tenside und Salze von gesättigten und/oder ungesättigten Fettsäuren), Lotionen und Cremes, Deodorantien, anderen wässrigen oder alkoholischen Lösungen, z.B. Reinigungslösungen für die Haut, feuchten Reinigungstüchern, Ölen oder Pudern.

Einen weiteren Erfindungsgegenstand bildet daher ein Körperpflegemittel, enthaltend mindestens eine Verbindung der Formel (1) sowie kosmetisch verträgliche Träger- oder Hilfsstoffe.

Das erfindungsgemässe Körperpflegemittel enthält 0,01 bis 15, vorzugsweise 0,1 bis 10 Gew.-%, bezogen auf das Gesamtgewicht der Zusammensetzung, der Verbindung der Formel (1) und kosmetisch verträgliche Hilfsstoffe.

Je nachdem, in welcher Form das Körperpflegemittel vorliegt, weist es neben dem Alkoxybenzylamin der Formel (1) noch weitere Bestandteile auf, wie z.B. Sequestriermittel, Farbstoffe, Parfümöle, Verdickungs- bzw. Festigungsmittel (Konsistenzregler), Emmollients, UV-Absorber, Hautschutzmittel, Antioxidantien, die mechanischen Eigenschaften verbessernde Additive wie Dicarbonsäuren und/oder Al-, Zn-, Ca-, Mg-Salze von C₁₄-C₂₂-Fettsäuren und gegebenenfalls Konservierungsmittel.

Das erfindungsgemässe Körperpflegemittel kann als Wasser-in-Öl- oder Öl-in-Wasser-Emulsion, als alkoholische oder alkoholhaltige Formulierung, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Erfindungsgemässe kosmetische Formulierungen werden in verschiedenen Bereichen eingesetzt. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-Ups in Form von Tages- oder Pudercremes, Gesichtspuder (lose und gepresst), Rouge oder Creme-Make-Ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspiranten oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Eine antimikrobielle Seife hat z.B. folgende Zusammensetzung:
0,01 bis 5 Gew.-% der Verbindung der Formel (1)
0,3 bis 1 Gew.-% Titandioxid,
1 bis 10 Gew.-% Stearinsäure
ad 100% Seifengrundlage, wie z.B. die Natriumsalze der Talgfett- und Kokosfettsäure oder Glycerine.

Ein Schampoo hat z.B. die folgende Zusammensetzung:
0,01 bis 5 Gew.-% der Verbindung der Formel (1),
12,0 Gew.-% Natrium-Laureth-2-sulfat,
4,0 Gew.-% Cocamidopropylbetain,
3,0 Gew.-% NaCI und
Wasser ad 100%.

Ein Deodorant hat z.B. die folgende Zusammensetzung:
0,01 bis 5 Gew.-% der Verbindung der Formel (1),
60 Gew.-% Ethanol,
0,3 Gew.-% Parfümöl, und
Wasser ad 100 %.

Einen weiteren Erfindungsgegenstand bildet eine orale Zusammensetzung, enthaltend 0,01 bis 15 Gew.-%, bezogen auf das Gesamtgewicht der Zusammensetzung, der Verbindung der Formel (1) und oral verträgliche Hilfsstoffe.

Beispiel für eine orale Zusammensetzung:
10 Gew.-% Sorbitol,
10 Gew.-% Glycerin,
15 Gew.-% Ethanol,
15 Gew.-% Propylenglykol,
0,5 Gew.-% Natriumlaurylsulfat,
0,25 Gew.-% Natriummethylcocyltaurat,
0,25 Gew.-% Polyoxypropylen/Polyoxyethylen-Blockcopolymer,
0,10 Gew.-% Pfefferminzgeschmacksstoff,
0,1 bis 0,5 Gew.-% einer Verbindung der Formel (1), und
48,6 Gew.-% Wasser.

Die erfindungsgemässe orale Zusammensetzung kann z.B. in Form eines Gels, einer Paste, einer Creme oder einer wässrigen Zubereitung (Mundwasser) vorliegen.

Weiterhin kann die erfindungsgemässe orale Zusammensetzung Verbindungen enthalten, die Fluoridionen freisetzen, die gegen die Bildung von Karies wirksam sind, z.B. anorganische Fluoridsalze, wie z.B. Natrium-, Kalium-, Ammonium- oder Calciumfluorid oder organische Fluoridsalze, wie z.B. Aminfluoride, die unter dem Handelsnamen Olafluor bekannt sind.

Weiterhin eignen sich die erfindungsgemässen Alkoxybenzylamine der Formel (1) für die Behandlung, insbesondere Konservierung von textilen Fasermaterialien. Es handelt sich dabei um ungefärbte und gefärbte oder bedruckte Fasermaterialien z.B. aus Seide, Wolle, Polyamid oder Polyurethanen, und insbesondere cellulosehaltige Fasermaterialien aller Art. Solche Fasermaterialien sind beispielsweise natürliche Cellulosefasern, wie Baumwolle, Leinen, Jute und Hanf, sowie Zellstoff und regenerierte Cellulose. Bevorzugte geeignete textile Fasermaterialien sind aus Baumwolle.

Die erfindungsgemässen Alkoxybenzylamine eignen sich auch zur Behandlung, insbesondere zur antimikrobiellen Ausrüstung oder Konservierung von Kunststoffen, wie z.B. Polyethylen, Polypropylen, Polyurethan, Polyester, Polyamid, Polycarbonat, Latex etc.. Einsatzbereiche dafür sind z.B. Fussbodenbeläge, Kunststoffbeschichtungen, Kunststoffbehälter- und Verpackungsmaterialien; Küchen- und Badezimmer-Utensilien (z.B. Bürsten, Duschvorhänge; Schwämme, Badezimmermatten), Latex, Filtermaterialien (Luft- und Wasserfilter), Kunststoffartikel, die im medizinischen Bereich eingesetzt werden, wie z.B. Verbandmaterialien, Spritzen, Katheter etc., sog. "medical devices", Handschuhe und Matratzen.

Auch Papier, wie z.B. Hygienepapiere können mit den erfindungsgemässen Alkoxybenzylamine antimikrobiell ausgerüstet werden.

Weiterhin können Nonwovens, wie z.B. Windeln, Damenbinden, Dameneinlagen, Tücher für den Hygiene- und Haushaltsbereich erfindungsgemäss antimikrobiell ausgerüstet werden. Weiterhin finden die Alkoxybenzylamine der Formel (1) Verwendung in Wasch- und Reinigungsformulierungen, wie z.B. in Flüssig- und Pulverwaschmitteln oder Weichspülern. Die Alkoxybenzylamine der Formel (1) können insbesondere auch in Haushalts- und Allzweckreinigern zur Reinigung und Desinfektion von harten Oberflächen eingesetzt werden.

Ein Reinigungsmittel hat z.B. folgende Zusammensetzung:

| | |
|---|---|
| 0,01 bis 5 % | der Verbindung der Formel (1) |
| 3,0 % | Octylalkohol 4EO |
| 1,3 % | Fettalkohol C₈-C₁₀-Polyglucosid |
| 3,0 % | Isopropanol |
| ad 100 % | Wasser. |

Neben der Konservierung von Kosmetik- und Haushaltsprodukten ist auch die Konservierung und antimikrobielle Ausrüstung von technischen Produkten sowie der Einsatz als Biozid in technischen Prozessen möglich, wie z.B. bei der Papierbehandlung, insbesondere in Papierbehandlungsflotten, Druckverdickern aus Stärke oder Celluloseabkömmlingen, Lacken und Anstrichfarben.

Die Alkoxybenzylamine der Formel (1) eignen sich auch zur antimikrobiellen Holzbehandlung sowie zur antimikrobiellen Behandlung, Konservierung und Ausrüstung von Leder.

Weiterhin eignen sich die erfindungsgemässen Verbindungen zum Schutz von kosmetischen Produkten und Haushaltsprodukten vor mikrobieller Verderbnis.

Die folgenden Beispiele veranschaulichen die vorliegende Erfindung, schränken diese aber nicht ein.

### Ausführungsbeispiele:

### Darstellung von Alkoxybenzylaminen

### Allgemeine Methode zur Darstellung von Alkoxybenzaldehyden durch Alkylierung von Hydroxybenzaldehyden:

43 mmol des Aldehyds werden in 60 ml Ethanol gelöst und mit 6.5 g (49 mmol) Kaliumcarbonat versetzt. Nach Zutropfen einer Lösung von 43 mmol des entsprechenden Bromalkans in 30 ml Ethanol wird 16 h unter Rückfluss erhitzt. Nach Abfiltrieren über wenig Kieselgel und Abziehen des Lösungsmittels wird der Rückstand in 400 ml tert.-Butylmethylether aufgenommen und die Lösung dreimal mit je 200 ml 1 m Natronlauge gewaschen. Die alkylierten Benzaldehyde werden nach dem Abziehen des Lösungsmittels ohne weitere Reinigung für die parallele Aminierung verwendet.

Aus 6.5 g Vanillin und 10 ml Bromdodecan werden so z.B. 10.0 g (73 % d.Th.) 3-Methoxy-4-dodecyloxy-benzaldehyd erhalten.

### Allgemeine Methode zur parallelen reduktiven Aminierung verschiedener alkoxylierter Benzaldehyde zu den Verbindungen der Formel (3) bis (102)

1 mmol des alkoxylierten Aldehyds wird in 2.5 ml Trimethylorthoformiat (TMOF) gelöst, und dann mit einer Lösung von 1 mmol des Amins in 2.5 ml TMOF und 0.19 ml Ameisensäure versetzt. Nach Zugabe von Molsieb (4A) wird das Gemisch 4 h bei 120°C gerührt. Nach Einengen der Reaktionsmasse wird das Produkt durch Chromatographie an Kieselgel (Eluent Ethanol/Triethylamin 10/1) gereinigt. Die Reinheit und Struktur der Verbindungen werden durch HPLC/MS kontrolliert bzw. bestätigt.
So werden z.B. durch Umsetzung von 3-Methoxy-4-dodecyloxy-benzaldehyd und Pyrrolidin 85 mg (23 % d.Th.) der Verbindung der Formel (4) erhalten (m/z = 375).

## Patentansprüche

1. Verbindunqen der Formel 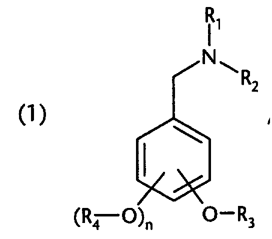 worin
R₁ und R₂ unabhängig voneinander Wasserstoff, nicht substituiertes oder mit einem oder mehreren Hydroxy, Halogen, C₁-C₁₈-Alkyl, C₁-C₁₈-Alkoxy oder Amino substituiertes C₁-C₂₀-Alkyl, C₂-C₂₀-Alkenyl, C₂-C₂₀-Alkinyl, C₃-C₁₂-Cycloalkyl, C₃-C₇-Cycloalkyl-C₁-C₂₀-Alkyl, C₁-C₆-Alkoxy-C₁-C₂₀-Alkyl, C₁-C₁₂-Alkyl-oxycarbonyl, Phenyl, Phenyl-C₁-C₂₀-Alkyl; Carboxy; oder Pyridino-C₁-C₅-alkyl; oder
R₁ und R₂ zusammen mit dem sie verbindenden Stickstoffatom einen 5- bis 7-gliedrigen monocyclischen heterocyclischen Ring bilden;
R₃ und R₄ unabhängig voneinander nicht substituiertes oder mit einem oder mehreren Hydroxy, Halogen, C₁-C₁₈-Alkyl, C₁-C₁₈-Alkoxy, oder Amino substituiertes C₁-C₂₀-Alkyl; und
n 0 oder 1;
bedeuten.

2. Verbindungen nach Anspruch 1, **dadurch gekennzeichnet, dass**
R₁ und R₂ unabhängig voneinander Wasserstoff; nicht substituiertes oder mit einem oder mehreren Hydroxy, Halogen, C₁-C₁₈-Alkyl, C₁-C₁₈-Alkoxy, Amino substituiertes C₁-C₂₀-Alkyl, Cyclo-C₅-C₇-Alkyl, Phenyl oder Phenyl-C₁-C₄-Alkyl; oder Pyridino-C₁-C₅-Alkyl;
bedeuten.

3. Verbindungen nach Anspruch 1 oder 2, **dadurch gekennzeichnet, dass**
R₁ und R₂ unabhängig voneinander C₁-C₂₀-Alkyl; Phenyl, Phenyl-C₁-C₄-Alkyl; oder Pyridino-C₁-C₄-Alkyl;
bedeuten.

4. Verbindungen nach einem der Ansprüche 1 bis 3, **dadurch gekennzeichnet, dass**
R₁ und R₂ unabhängig voneinander C₁-C₁₂-Alkyl; Benzyl; oder Pyridinoethyl;
bedeuten.

5. Verbindungen nach Anspruch 1, **dadurch gekennzeichnet, dass**
R₁ und R₂ zusammen mit dem sie verbindenden Stickstoffatom einen nicht weiter substituierten oder mit einem oder mehreren C₁-C₅-Alkyl substituierten -(CH₂)₂₋₆- -Rest, der gegebenenfalls mit einem oder 2 -O- und/oder -NR'- -Gruppen und/oder - 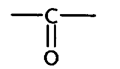 unterbrochen ist;
R' Wasserstoff; nicht substituiertes oder mit einem oder mehreren Hydroxy, Halogen, C₁-C₁₈-Alkyl, C₁-C₁₈-Alkoxy, Amino oder quaternären Ammoniumgruppen substituiertes C₁-C₁₈-Alkyl, Cyclo-C₅-C₇-Alkyl oder Phenyl; -COR"; und
R" Wasserstoff; oder C₁-C₄-Alkyl;
bedeuten.

6. Verbindungen nach Anspruch 5, **dadurch gekennzeichnet, dass**
R' Wasserstoff; C₁-C₅-Alkyl; einen Rest der Formel 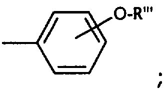 oder einen Rest der Formel 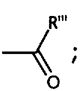 und
R''' Wasserstoff; oder C₁-C₅-Alkyl;
bedeuten.

7. Verbindungen nach einem der Ansprüche 1 bis 6, **dadurch gekennzeichnet, dass**
R₃und R₄ unabhängig voneinander C₁-C₁₈-Alkyl bedeuten.

8. Verbindungen nach Anspruch 7, **dadurch gekennzeichnet, dass**
R₃ und R₄ die gleiche Bedeutung haben.

9. Verbindungen nach Anspruch 7, **dadurch gekennzeichnet, dass**
R₃ C₃-C₁₂-Alkyl bedeutet.

10. Verbindungen nach Anspruch 7, **dadurch gekennzeichnet, dass**
R₄ Methyl
bedeutet.

11. Verbindungen nach einem der Ansprüche 1 bis 10, **dadurch gekennzeichnet, dass** sie der Formel 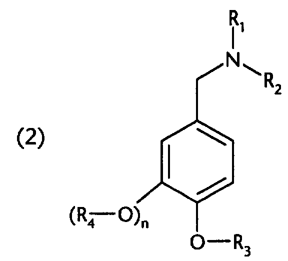 entsprechen, worin
R₁, R₂, R₃, R₄ und n die in Anspruch (1) angegebene Bedeutung haben.

12. Verbindungen nach Anspruch 11, **dadurch gekennzeichnet, dass**
R₁ und R₂ zusammen mit dem sie verbindenden Stickstoffatom einen nicht weiter substituierten oder mit einem oder mehreren C₁-C₅-Alkyl substituierten -(CH₂)₂₋₆- -Rest, der gegebenenfalls mit einem oder 2 -O- und/oder -NR'- -Gruppen und/oder - 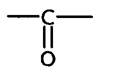 unterbrochen ist;
R' Wasserstoff; nicht substituiertes oder mit einem oder mehreren Hydroxy, Halogen, C₁-C₁₈-Alkyl, C₁-C₁₈-Alkoxy, Amino oder quaternären Ammoniumugruppen substituiertes C₁-C₁₈-Alkyl, Cyclo-C₅-C₇-Alkyl oder Phenyl; -COR"; und
R" Wasserstoff; oder C₁-C₄-Alkyl;
R₃ und R₄ unabhängig voneinander C₁-C₁₈-Alkyl; und
n 0; oder 1;
bedeuten.

13. Verbindungen nach Anspruch 11, **dadurch gekennzeichnet, dass**
R₁ und R₂ unabhängig voneinander C₁-C₂₀-Alkyl; Phenyl; Phenyl-C₁-C₄-Alkyl; oder Pyridino-C₁-C₄-Alkyl;
R₃ C₃-C₁₂-Alkyl; und
R₄ C₁-C₁₂-Alkyl, insbesondere Methyl;
bedeuten.

14. Verfahren zur Herstellung der Verbindungen der Formel (1) durch Alkylierung eines Hydroxybenzaldehyds (wenn R₄ Wasserstoff bedeutet) bzw. eines Alkyloxyhydroxyaldehyds oder eines Dihydroxybenzaldehyds mit einem Alkylhalogenid in einem geeigneten Lösungsmittel unter Verwendung einer Hilfsbase (1. Reaktionsschritt) und Kondensation des erhaltenen Mono- bzw. Bisalkoxybenzaldehyd der Formel (1d) zur Verbindung der Formel (1) in einem geeigneten Lösungsmittel oder in einem Überschuss eines sekundären Amins ohne Lösungsmittel mittels eines wasserentziehenden Agens oder durch azeotrope Destillation von Wasser mit einem sekundären Amin und gleichzeitiger Zugabe eines Reduktionsmittels (2. Reaktionsschritt) nach folgendem Reaktionsschema:

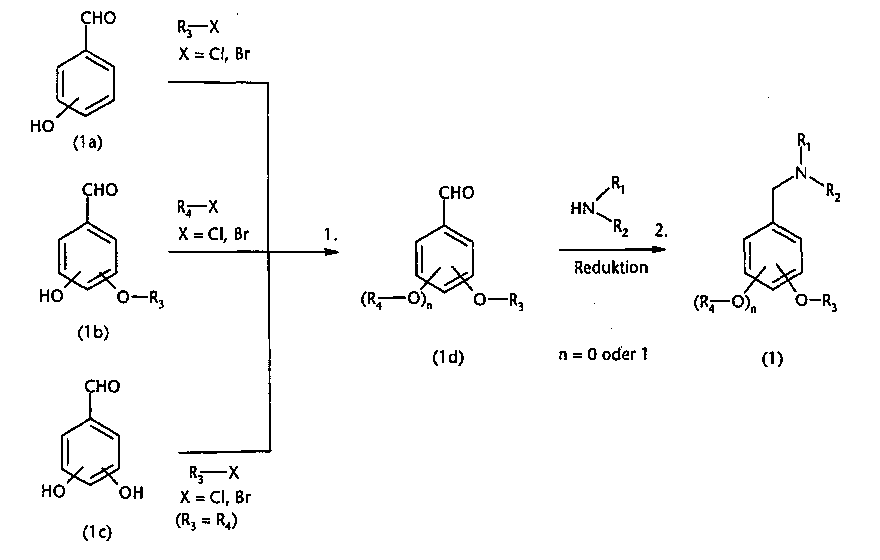

15. Verwendung der Verbindungen der Formel (1) nach Anspruch 1 zur antimikrobiellen Behandlung von Oberflächen.

16. Verwendung der Verbindung der Formel (1) zur antimikrobiellen Behandlung, Desodorierung und Desinfektion der Haut, Schleimhäute und Haare.

17. Verwendung nach Anspruch 16, **dadurch gekennzeichnet, dass** die Verbindung der Formel (1) zur Desinfektion und Desodorierung verwendet wird.

18. Verwendung der Verbindung der Formel (1) zur Behandlung von textilen Fasermaterialien.

19. Verwendung nach Anspruch 18, **dadurch gekennzeichnet, dass** die Verbindung der Formel (1) zur Konservierung verwendet wird.

20. Verwendung der Verbindung der Formel (1) in Wasch- und Reinigungsformulierungen.

21. Verwendung der Verbindung der Formel (1) zur antimikrobiellen Ausrüstung und Konservierung von Kunststoffen, Papier, Nonwovens, Holz oder Leder.

22. Verwendung der Verbindung der Formel (1) zur antimikrobiellen Ausrüstung und Konservierung von technischen Produkten, insbesondere Druckverdickern aus Stärke oder Celluloseabkömmlingen, Lacken und Anstrichfarben.

23. Verwendung der Verbindung der Formel (1) als Biozid in technischen Prozessen.

24. Körperpflegemittel, enthaltend
0,01 bis 15 Gew.-%, bezogen auf das Gesamtgewicht der Zusammensetzung, der Verbindung der Formel (1) und kosmetisch verträgliche Hilfsstoffe.

25. Orale Zusammensetzung, enthaltend 0,01 bis 15 Gew.-%, bezogen auf das Gesamtgewicht der Zusammensetzung, der Verbindung der Formel (1) und oral verträgliche Hilfsstoffe.
